# EUROPÄISCHE PATENTSCHRIFT

(11) **EP 3 218 494 B1**
(45) Veröffentlichungstag und Bekanntmachung des Hinweises auf die Patenterteilung: **30.10.2024**
(21) Anmeldenummer: 15797045.0
(22) Anmeldetag: 12.11.2015
(51) Int. Cl.: A01H 1/08, C07K 14/415, C12N 9/10, C12N 9/16, C12N 9/18, C12N 9/90, C12N 15/82

(54) **HAPLOIDENINDUKTOREN**
HAPLOID INDUCERS
INDUCTEURS HAPLOÏDES

(30) Priorität: 12.11.2014 DE 102014016667
(43) Veröffentlichungstag der Anmeldung: 20.09.2017
(73) Patentinhaber: KWS SAAT SE & Co. KGaA, 37574 Einbeck (DE)
(72) Erfinder: BOLDUAN, Christof, 37574 Einbeck (DE); KLOIBER-MAITZ, Monika, 37574 Einbeck (DE); NIESSEN, Markus, 30167 Hannover (DE); OUZUNOVA, Milena, 37077 Göttingen (DE); WELTMEIER, Fridtjof, 37574 Einbeck (DE)
(86) Internationale Anmeldenummer: PCT/EP2015/076469
(87) Internationale Veröffentlichungsnummer: WO 2016/075255

(56) Entgegenhaltungen:
- WO-A1-2010/083178
- WO-A1-2011/044132
- WO-A1-2013/072832
- WO-A1-2014/151749
- WO-A1-2016/177887
- WO-A2-2014/110274
- RÖBER F K ET AL: "In vivo haploid induction in maize - Performance of new inducers and significance of doubled haploid lines in hybrid breeding", MAYDICA, ISTITUTO SPERIMENTALE PER LA CEREALICOLTURA, BERGAMO, IT, vol. 50, 1 January 2005 (2005-01-01), pages 275 - 283, XP002508662, ISSN: 0025-6153
- DATABASE Geneseq [online] 6 November 2014 (2014-11-06), "Zea mays target gene (yield) encoded protein, SEQ ID:3454.", XP002752624, retrieved from EBI accession no. GSP:BBO35816 Database accession no. BBO35816
- DATABASE Geneseq [online] 6 November 2014 (2014-11-06), "Zea mays agronomic trait (yield) target gene, SEQ ID:2123.", XP002752625, retrieved from EBI accession no. GSN:BBO34485 Database accession no. BBO34485
- DATABASE Geneseq [online] 18 July 2013 (2013-07-18), "Zea mays growth related protein (GRP) polypeptide, SEQ:496.", XP002752626, retrieved from EBI accession no. GSP:BAO59625 Database accession no. BAO59625
- MARUTHACHALAM RAVI & SIMON W L CHAN: "Haploid plants produced by centromere-mediated genome elimination", NATURE, NATURE PUBLISHING GROUP, UNITED KINGDOM, vol. 464, no. 7288, 25 March 2010 (2010-03-25), pages 615 - 620, XP002677783, ISSN: 0028-0836, DOI: 10.1038/NATURE08842
- H. J. KIM ET AL: "Endoplasmic Reticulum- and Golgi-Localized Phospholipase A2 Plays Critical Roles in Arabidopsis Pollen Development and Germination", THE PLANT CELL, vol. 23, no. 1, 1 January 2011 (2011-01-01), US, pages 94 - 110, XP055238464, ISSN: 1040-4651, DOI: 10.1105/tpc.110.074799
- DONG X ET AL: "Fine mapping of qhir1 influencing in vivo haploid induction in maize", THEORETICAL AND APPLIED GENETICS, SPRINGER, BERLIN, DE, vol. 126, no. 7, 29 March 2013 (2013-03-29), pages 1713 - 1720, XP035333157, ISSN: 0040-5752, [retrieved on 20130329], DOI: 10.1007/S00122-013-2086-9

## Beschreibung

### Gebiet der Erfindung

Die vorliegende Erfindung betrifft das Gebiet der Modifikation von Pflanzen mittels molekularbiologischer Methoden und Markertechnologie und der Gentechnik. Sie bezieht sich die Bereitstellung von technischen Mitteln wie Nukleinsäuren und Vektoren sowie Verfahren und Verwendungen zur Herstellung und zum Identifizieren von nicht-transgenen und transgenen pflanzlichen Haploiden-Induktoren wie auch die Verbesserung vorhandener pflanzlicher Haploiden-Induktoren.

### Hintergrund der Erfindung

Bei der Herstellung von hybriden Pflanzen werden üblicherweise zwei Inzuchtlinien als Eltern miteinander gekreuzt, deren Nachkommen aufgrund des bekannten Heterosis-Effekts einen gegenüber den Elternlinien teils stark erhöhten Ertrag generieren. Die Inzuchtlinien können durch mehrere Selbstungsschritte erhalten werden, was jedoch mehrere Generationen in Anspruch nimmt und damit mit einem enormen Zeitaufwand verbunden ist. Schon vor vielen Jahren ging man in der moderenen Pflanzenzüchtung dazu über vermehrt die Inzuchtlinien durch Haploideninduktion und anschließender Chromosomenverdopplung in sehr viel kürzerer Zeit zu erzeugen. Technische Voraussetzung hierfür ist ein funtionierendes - Haploideninduktionsystem, welches zugleich auch eine ausreichende Effizienz verspricht, um wirtschaftlich einsetzbar zu sein.

Für Mais (*Zea mays*) ist beispielsweise ein maternales *in vivo* Induktionssystem bekannt, bei welchem die zu induzierende Pflanze mit Pollen des Induktors bestäubt wird. Bis zu 10% der hierbei erzeugten Nachkommen enthalten dann nur den einfachen (haploiden) Chromosomensatz des Saatelter. Der Mais-Hybridzüchtung stehen heute einige derartige Induktoren zur Verfügung. Jedoch sind diese alle auf die eine Linie "Stock 6", beschrieben in Coe, 1959, zurückzuführen. Ein Beispiel für einen solchen bekannten Induktor ist die Linie RWS (Röber et al, 2005). In der Vergangenheit wurden an diesen Linien mehrere QTL-Studien zur Identifikation der induktorrelevanten Loci durchgeführt. Bereits 1997 wurde von Deimling et al. ein Haupt-QTL auf Chromosom 1 (bin 1.04) im Mais identifiziert. Genauere Kartierung erfolgte durch Barret et al. 2008 auf den Bereich zwischen 66,96 MB und 68,11 MB auf Chromosom 1, von Prigge et al. 2012 auf den Bereich zwischen 62,9 MB und 70,8 MB, und daran anschließend von Dong et al. 2013 auf den Bereich zwischen 68,18 MB und 68,43 MB, welche laut öffentlicher Annotation drei Gene enthält. Alle Positionsangaben beziehen sich auf das Referenzgenom von B73, Version AGPv02. Die Funktionalität des Locus für sich alleine scheint von Dong et al. 2014 durch Erreichen einer Induktionsrate von 5% bereits nachgewiesen zu sein. Jedoch kann eine fehlerhafte Feinkartierung nicht ausgeschlossen werden, da mangels der Angabe flankierender Marker im rekurrenten Elter keine eindeutige Abgrenzung des QTLs möglich ist.

Weiterhin offenbart WO 2012/030893 einen induktorrelevanten Locus auf Chromosom 1 in Mais, der sich aber deutlich von vorstehendem Locus unterscheidet und näher am Telomer lokalisiert ist. Es gibt keine Überlappung der betrachteten Genomregionen.

Insgesamt sind die molekularen und entwicklungsspezifischen Mechanismen, welche die *in vivo*-Haploideninduktion bei Maislinien, welche aus "Stock 6" hervorgegangen sind, bis heute weitgehend unbekannt. Denkbar ist beispielsweise, dass eine Befruchtung stattfindet, es aber anschließend zu einer Chromosomenelimination kommt, welche dann haploide Nachkommen entstehen lässt. Ein solcher Mechanismus ist beispielsweise in einem System mit dem Histonprotein CenH3 von Ravi & Chan (2010) beschrieben worden. Andererseits kann aber auch die Befruchtung ausbleiben und die Entwicklung der haploiden Eizelle findet im triploiden Endosperm statt. Ohne das Verständnis für die zugrundeliegende maternale *in vivo-*Haploideninduktionseignung eines Induktorgenotyps abgeleitet von "Stock 6" und das Wissen über die verantwortlichen Gene ist eine gezielte Verbesserung dieses Mais-Induktorgenotyps oder die Übertragung der Induktionseignung auf Nicht-Induktorgenotypen beziehungsweise das gezieltes Herbeiführen der *in vivo*-Haploiden-Induktionsleistung in Mais-Nicht-Induktoren praktisch unmöglich.

Weiterhin ist für einige Kulturpflanzen überhaupt kein effizientes und damit wirtschaftlich anwendbares System zur Herstellung haploider und doppelhaploider Pflanzen bekannt, wie z.B für Sorghum, Roggen oder Sonnenblume.

Es besteht also ein Bedarf an der Bereitstellung von genetischen Elementen, wie Gene oder regulatorische Elemente, die in transgenen und/oder nicht-transgenen Ansätzen einsetzbar sind, um über in vivo-Induktion die Haploidenentwicklung oder eine verbesserte Effizienz bei der Haploidenentwicklung zu ermöglichen.

### Zusammenfassung der Erfindung

Die vorliegende Erfindung wurde vor dem Hintergrund des vorstehend beschriebenen Stands der Technik gemacht, wobei es Aufgabe der vorliegenden Erfindung ist, Mittel und Verfahren bereitzustellen, welche zur Herstellung eines in vivo-haploiden Induktors und/oder zur Herstellung einer haploiden Pflanze eingesetzt werden können.

Erfindungsgemäß erfolgt die Lösung der gestellten Aufgabe mittels einer Nukleinsäure, welche nach Transkription oder nach Expression in einer Pflanze geeignet ist, die Eigenschaft eines Haploideninduktors zu vermitteln oder die Induktionsleistung eines Haploideninduktors zu erhöhen. Die erfindungsgemäße Nukleinsäure kann als Transgen eingesetzt werden. Andererseits kann auch eine endogene DNA-Sequenz im Genom einer Pflanze oder im Genom eines pflanzlichen Haploideninduktors, welche identisch ist mit einer der erfindungsgemäßen Nukleinsäuren, derart modifiziert werden, dass in der Pflanze nach Transkription oder nach Expression der endogenen DNA-Sequenz die Eigenschaft eines Haploideninduktors vermittelt wird oder die Induktionsleistung des Haploideninduktors erhöht wird. Vorzugsweise handelt es sich bei der Nukleinsäure der vorliegenden Erfindung um eine isolierte Nukleinsäure, welche aus ihrer natürlichen bzw. ursprünglichen Umgebung (genetischer Kontext) herausgelöst ist. Eine Nukleinsäure kann doppelstängig oder einsträngig, linear oder zirkulär sein. Es kann sich hierbei um genomische DNA, synthetische DNA, cDNA oder einen RNA-Typ (beipielsweise IncRNA, siRNA oder miRNA) handeln, wobei in RNA statt der Nukleobase Thymin die Nukleobase Uracil vorkommt.

In einer bevorzugten Ausgestaltung der vorliegenden Erfindung hat die erfindungsgemäße Nukeinsäure oder eine von der Nukleinsäure kodierte RNA oder ein von der Nukleinsäure kodiertes Protein oder Polypeptid Einfluss auf das Pollenschlauch-Wachstum in einer Pflanze, auf den Energiestoffwechsel eines Pollens einer Pflanze und/oder auf die Aktivität des Centromers vorzugsweise in einer generativen Zelle, welche sich beispielsweise zu einem Pollen entwickelt.

Die erfindungsgemäße Nukeinsäure kann sich dadurch auszeichnen, dass die Nukleinsäure oder eine von der Nukleinsäure kodierte RNA oder ein von der Nukleinsäure kodiertes Protein oder Polypeptid geeignet ist bzw. eingesetzt werden kann, um beispielsweise in einem Pollen einer Pflanze das Pollenschlauch-Wachstum im Vergleich mit einem Pollen einer Wildtyp-Pflanze, in welcher die erfindungsgemäße Nukleinsäure oder eine von der Nukleinsäure kodierte RNA oder ein von der Nukleinsäure kodiertes Protein oder Polypeptid nicht wie nachfolgend beschrieben verwendet wird, zu beschleunigen oder zu fördern. Die erfindungsgemäße Nukleinsäure kodiert beispielsweise für Enzyme der Klasse der Phospoholipasen, insbesondere der Phospholipase A2 oder der Patatin-Phospholipase, in der Lage das Wachstum des Pollenschlauchs zu fördern (Kim et al., 2011). Die erfindungsgemäßen Nukleinsäure kann zum Zweck des beschleunigten Pollenschlauchwachstums als Transgen eingesetzt wird, wobei sie dann zum Beispiel mittels Überexpressionsansatz die Expressionsrate eines Pollenschlauchwachstumsfördernden Gens oder die Transkriptionsrate einer RNA wie einer IncRNA, welche ein Pollenschlauchwachstumsfördernden Gene positiv reguliert (aktiviert) oder Pollenschlauchwachstumshemmende Gene negativ reguliert (inhibiert), in einer Pflanze oder einem Teil davon im Vergleich zu einer Wildtyp-Pflanze oder entsprechenden Teil davon erhöht und/oder mittels RNAi-Ansatz oder miRNA-Ansatz (Fire et al., 1998) die Expressionsrate eines Pollenschlauchwachstumshemmenden Gens, in einer Pflanze oder einem Teil davon im Vergleich zu einer Wildtyp-Pflanze oder entsprechenden Teil davon reduziert ist. Andererseits kann auch eine endogene DNA-Sequenz im Genom einer Pflanze oder im Genom eines pflanzlichen Haploideninduktors, welche identisch mit der erfindungsgemäßen Nukleinsäure ist, oder eine regulatorische Sequenz der endogenen DNA-Sequenz zum Beispiel durch Mutagenisieren oder 'Genome Editing' modifiziert werden. Diese Modifikation kann die Transkriptions- oder Expressionsrate der endogenen DNA-Sequenz oder die Aktivität oder Stabilität des durch die endogene DNA-Sequenz kodierten Proteins oder Polypeptids in einer Pflanze im Vergleich zu einer nicht-mutagenisierten Wildtyp-Pflanze erhöhen oder reduzieren. Somit kann beispielsweise die Expressionsrate eines endogenen Pollenschlauchwachstumsfördernden Gens oder die Transkriptionsrate einer endogenen RNA wie einer IncRNA, welche ein Pollenschlauchwachstumsförderndes Gen positiv reguliert (aktiviert) oder ein Pollenschlauchwachstumshemmendes Gen negativ reguliert (inhibiert), in einer Pflanze im Vergleich zu einer nicht-mutagenisierten oder über 'Genome Editing' modifizierten Wildtyp-Pflanze erhöht werden oder die Expressionsrate eines endogenen Pollenschlauchwachstumshemmenden Gens oder die Transkriptionsrate einer RNA wie einer IncRNA, welche ein Pollenschlauchwachstumsförderndes Gen negativ reguliert (inhibiert) oder ein Pollenschlauchwachstumshemmendes Gen positiv reguliert (aktiviert), in einer Pflanze im Vergleich zu einer nicht-mutagenisierten oder über 'Genome Editing' modifizierten Wildtyp-Pflanze reduziert werden. Außerdem kann die Aktivität oder Stabilität eines durch die endogene DNA-Sequenz kodierten Pollenschlauchwachstumsfördernden Proteins oder Polypeptids in einer Pflanze im Vergleich zu einer nicht-mutagenisierten oder über 'Genome Editing' modifizierten Wildtyp-Pflanze erhöht oder die Aktivität oder Stabilität eines durch die endogene DNA-Sequenz kodierten Pollenschlauchwachstumshemmenden Proteins oder Polypeptids in einer Pflanze im Vergleich zu einer nicht-mutagenisierten oder über 'Genome Editing' modifizierten Wildtyp-Pflanze reduziert werden.

In einem weiteren Beispiel kann die erfindungsgemäße Nukeinsäure sich dadurch auszeichnen, dass durch die Verwendung der Nukleinsäure oder einer von der Nukleinsäure kodierten RNA oder eines von der Nukleinsäure kodierten Proteins oder Polypeptids der Energiestoffwechsel eines Pollens in einer Pflanze im Vergleich mit einer Wildtyp-Pflanze beeinträchtigt werden. Dies kann beispielsweise durch eine Phosphoglycerat-Mutase erfolgen. Die erfindungsgemäße Nukleinsäure kann zu diesem Zweck als Transgen im Überexpressionsansatz oder im RNAi-Ansatz oder miRNA-Ansatz (Fire et al., 1998) eingesetzt wird. Andererseits kann auch eine endogene DNA-Sequenz im Genom einer Pflanze oder im Genom eines pflanzlichen Haploideninduktors, welche identisch mit der erfindungsgemäßen Nukleinsäure ist, oder eine regulatorische Sequenz der endogenen DNA-Sequenz zum Beispiel durch Mutagenisieren oder 'Genome Editing' modifiziert werden. Diese Modifikation kann die Transkriptions- oder Expressionsrate der endogenen DNA-Sequenz oder die Aktivität oder Stabilität des durch die endogene DNA-Sequenz kodierten Proteins oder Polypeptids in der Pflanze im Vergleich zu einer nicht-mutagenisierten oder über 'Genome Editing' modifizierten Wildtyp-Pflanze erhöhen oder reduzieren.

In einem anderen Beispiel kann auch die erfindungsgemäße Nukeinsäure sich dadurch auszeichnen, dass durch die Verwendung der Nukleinsäure oder einer von der Nukleinsäure kodierten RNA oder eines von der Nukleinsäure kodierten Proteins oder Polypeptids die Aktivität des Centromers in einer Pflanze, insbesondere in der frühen Embryogenese und vorzugsweise in einer generativen Zelle der Pflanze, welche sich beispielsweise zu einem Pollen entwickelt, im Vergleich zu einer Wildtyp-Pflanze verändert ist, was beispielsweise zur Eliminierung des induktor-Genoms führen kann. Die Aktivität des Centromers kann über die Chomatinmodifikation auf DNA oder Histonebene, außerdem auch durch Transkription, RNA-Wechselwirkungen oder RNA-Bindung geändert werden. Eine Änderung der Aktivität des Centromers kann beispielsweise durch eine Methyltransferase wie eine RNA-Methyltransferase erfolgen. Die erfindungsgemäßen Nukleinsäure kann zu diesem Zweck als Transgen eingesetzt wird, wobei sie dann zum Beispiel mittels Überexpressionsansatz die Expressionsrate eines Chromatin-modifizierenden Gens oder die Transkriptionsrate einer RNA wie einer IncRNAs, welche ein Chromatin-modifizierendes Gen positiv reguliert (aktiviert), in einer Pflanze im Vergleich zu einer Wildtyp-Pflanze erhöht. Andererseits kann auch eine endogene DNA-Sequenz im Genom einer Pflanze oder im Genom eines pflanzlichen Haploideninduktors, welche identisch mit der erfindungsgemäßen Nukleinsäure ist, oder eine regulatorische Sequenz der endogenen DNA-Sequenz zum Beispiel durch Mutagenisieren oder 'Genome Editing' modifiziert werden. Diese Modifikation kann die Transkriptions- oder Expressionsrate der endogenen DNA-Sequenz oder die Aktivität oder Stabilität des durch die endogene DNA-Sequenz kodierten Proteins oder Polypeptids in einer Pflanze im Vergleich zur nicht-mutagenisierten oder über 'Genome Editing' modifizierten Wildtyp-Pflanze erhöhen oder reduzieren. Somit kann auch die Expressionrate eines endogenen Chromatin-modifizierenden Gens oder die Transkriptionsrate einer endogenen RNA wie einer IncRNA, welche ein Chromatin-modifizierendes Gen positiv reguliert (aktiviert), in einer Pflanze im Vergleich zur nicht-mutagenisierten oder nicht durch 'Genome Editing' modifizierten Wildtyp-Pflanze erhöht werden. Außerdem kann die Aktivität oder Stabilität eines durch die endogene DNA-Sequenz kodierten Chromatin-modifizierenden Proteins in einer Pflanze im Vergleich zur nicht-mutagenisierten oder nicht durch 'Genome Editing' modifizierten Wildtyp-Pflanze erhöht werden.

Vorstehend ausgeführte Verwendungen der erfindungsgemäßen Nukleinsäure oder einer von der Nukleinsäure kodierten RNA oder einem von der Nukleinsäure kodierten Protein oder Polypeptid sind nicht abschließend oder beschränkend, sondern nur beispielhaft zu verstehen. Einem Fachmann ist aus dem Stand der Technik zahlreiche weitere technische Mittel und Verfahren bekannt, mit welchen er oben beschriebene Änderungen der Expressions- oder Transkriptionsrate der erfindungsgemäßen Nukleinsäure oder der identischen endogenen DNA-Sequenz oder oben beschriebene Änderungen der Stabilität und Aktivität des durch die erfindungsgemäße Nukleinsäure oder die endogene DNA-Sequenz kodierten Proteins oder Polypeptids bewirken kann.

In einer besonders bevorzugten Ausgestaltung der vorliegenden Erfindung kann die Nukleinsäure, welche nach Transkription oder nach Expression in einer Pflanze geeignet ist, die Eigenschaft eines Haploideninduktors zu vermitteln oder die Induktionsleistung eines Haploideninduktors zu erhöhen, eine Nukleinsäure sein, die eine Nukleotidsequenz umfasst, die
(i) eine Sequenz ausgewählt aus SEQ ID NOs: 9 aufweist, oder
(ii) zu einer Sequenz aus (i) komplementär ist, oder
(iii) mit einer Sequenz aus (i) zu mindestens 90%, 91%, 92%, 93%, 94%, 95%, 96%, oder besonders bevorzugt mindestens 97%, 97,5%, 98%, 98,5%, 99% oder 99,5% identisch ist, oder
(iv) für ein Protein mit der Aminosäuresequenz ausgewählt aus SEQ ID NOs: 23.

Diese Nukleinsäure kann für ein Protein oder einen funktionellen Teil davon kodieren, wobei das Protein oder der funktionelle Teil davon die Funktionalität einer Phospoholipase, insbesondere einer Phospholipase A2 oder einer Patatin-Phospholipase, innehat (siehe Tabelle 1). Eine Verwendung der Nukleinsäure kann wie oben beschrieben erfolgen, d.h. um in einer Pflanze die Eigenschaft eines Haploideninduktors zu vermitteln oder die Induktionsleistung eines Haploideninduktors zu erhöhen, ist beispielsweise die Expressionsrate der Nukleinsäure oder die Aktivität oder Stabilität des kodierten Proteins oder des kodierten Teil des Proteins transgen oder endogen zu erhöhen. Da diese Nukleinsäure bzw. eine von der Nukleinsäure kodierte RNA oder ein von der Nukleinsäure kodiertes Protein oder Polypeptid eine positive Wirkung auf die Hapolideninduktionsleistung einer Pflanze haben, wird im Folgenden eine Nukleinsäure, welche hier definiert ist, als induktionsfördernde Nukleinsäure bezeichnet. Weitere Verfahren und Verwendungen der induktionsfördernen Nukleinsäure sowie Gegenstände, welche die induktionsfördernen Nukleinsäure umfassen, sind weiter unten offenbart.

In einer weiteren besonders bevorzugten Ausgestaltung der vorliegenden Erfindung kann die Nukleinsäure, welche nach Transkription oder nach Expression in einer Pflanze geeignet ist, die Eigenschaft eines Haploideninduktors zu vermitteln oder die Induktionsleistung eines Haploideninduktors zu erhöhen, eine Nukleinsäure sein, die für eine RNA kodiert, die einen doppelsträngigen Teil aufweist, wobei mindestens ein Strang des doppelsträngigen Teils eine Nukleotidsequenz aufweist, welche mit mindestens 14, 15, 16, 17, 18, 19, 20, 21, 22, 23, 24 oder 25, bevorzugt mit mindestens 30, 35, 40, 45, 50, 60, 70, 80, 90, 100, 120 oder 140, besonders bevorzugt mit mindestens 160, 180, 200, 250, 300, 350, 400, 450, 500, 600, 700, 800, 900 oder 1000 aufeinanderfolgenden Nukleotiden einer Intron-Sequenz einer Nukleinsäure, die
(i) eine Sequenz ausgewählt aus SEQ ID NOs: 9 oder ein Fragment davon in Sinn- oder Antisinnorientierung aufweist, oder
(ii) zu einer Sequenz aus (i) komplementär ist, oder
(iii) mit einer Sequenz aus (i) zu mindestens 90%, 91%, 92%, 93%, 94%, 95% oder 96%, oder besonders bevorzugt mindestens 97%, 97,5%, 98%, 98,5%, 99% oder 99,5% identisch ist, oder
(iv) für ein Protein mit der Aminosäuresequenz ausgewählt aus SEQ ID NOs: 23 oder einen Teil des Proteins kodiert

**Tabelle 1: Sequenzverzeichnis und Sequenzzuordnung der Nukleotid- und Animosäuresequenzen. Die Namen der Genfamilien/Proteinfamilien entsprechen den öffentlichen Modellen. In den Induktoren kann es durch strukturelle Änderungen zu abweichenden Funktionalitäten kommen.**

| SEQ ID NO: | Genfamilie/Proteinfamilie - Identifier // abgeleitetes Genmodel | Sequenztyp | Quelle |
|---|---|---|---|
| 1 | GRMZM2G179789 SNAREv 1 | genomisch DNA | B73 |
| 2 | GRMZM2G179789 SNAREv 1 | cDNA | B73 |
| 3 | GRMZM2G179789 SNAREv 1 | cDNA (Variante 1) | RWS |
| 4 | GRMZM2G179789 SNAREv 1 | cDNA (Variante 2) | RWS |
| 5 | GRMZM2G179789 SNAREv 1 | cDNA (Teilsequenz von Nukleotid 1240 bis 2321 der SEQ ID NO: 3) | RWS |
| 6 | GRMZM2G412426 SNAREv 2 | genomisch DNA | B73 |
| 7 | GRMZM2G412426 SNAREv 2 | cDNA | B73 |
| 8 | GRMZM2G471240 Patatin-Phospholipase | genomisch DNA inklusive Flanken | B73 |
| 9 | GRMZM2G471240 Patatin-Phospholipase | genomisch DNA inklusive Flanken | RWS |
| 10 | GRMZM2G471240 Patatin-Phospholipase | cDNA | B73 |
| 11 | GRMZM2G471240 Patatin-Phospholipase | cDNA | RWS |
| 12 | GRMZM2G347808 RNA-Methyltransferase | genomisch DNA inklusive Flanken | B73 |
| 13 | GRMZM2G347808 RNA-Methyltransferase | genomisch DNA inklusive Flanken | RWS |
| 14 | GRMZM2G347808 RNA-Methyltransferase | cDNA | B73 |
| 15 | GRMZM2G347808 RNA-Methyltransferase | cDNA | RWS |
| 16 | GRMZM2G179789 SNAREv 1 | Polypeptid (Variante 1) | B73 |
| 17 | GRMZM2G179789 SNAREv 1 | Polypeptid (Variante 2) | B73 |
| 18 | GRMZM2G179789 SNAREv 1 | Polypeptid (Variante 1) | RWS |
| 19 | GRMZM2G179789 SNAREv 1 | Polypeptid (Variante 2) | RWS |
| 20 | GRMZM2G412426 SNAREv 2 | Polypeptid | B73 |
| 21 | GRMZM2G471240 Patatin-Phospholipase | Polypeptid (Variante 1) | B73 |
| 22 | GRMZM2G471240 Patatin-Phospholipase | Polypeptid (Variante 2) | B73 |
| 23 | GRMZM2G471240 Patatin-Phospholipase | Polypeptid | RWS |
| 24 | GRMZM2G347808 RNA-Methyltransferase | Polypeptid | B73 |
| 25 | GRMZM2G347808 RNA-Methyltransferase | Polypeptid | RWS |
| 26 | GRMZM2G106834 Phosphoinositolphosphatase | genomisch DNA | B73 |
| 27 | GRMZM2G106834 Phosphoinositolphosphatase | cDNA (Variante 1) | B73 |
| 28 | GRMZM2G106834 Phosphoinositolphosphatase | cDNA (Variante 2) | B73 |
| 29 | GRMZM2G106834 Phosphoinositolphosphatase | cDNA (Variante 3) | B73 |
| 30 | GRMZM2G062320 Phosphoglycerat-Mutase | genomisch DNA | B73 |
| 31 | GRMZM2G062320 Phosphoglycerat-Mutase | cDNA | B73 |
| 32 | GRMZM2G106834 Phosphoinositolphosphatase | Polypeptid (Variante 1) | B73 |
| 33 | GRMZM2G106834 Phosphoinositolphosphatase | Polypeptid (Variante 2) | B73 |
| 34 | GRMZM2G062320 Phosphoglycerat-Mutase | Polypeptid | B73 |
| 35 | IncRNA zur Phosphoinositolphosphatase | cDNA (Variante 1) | B73 |
| 36 | IncRNA zur Phosphoinositolphosphatase | cDNA (Variante 2) | B73 |
| 37 | IncRNA zur Phosphoinositolphosphatase | cDNA (Variante 3) | B73 |
| 38 | IncRNA zur Phosphoinositolphosphatase | cDNA | RWS |
| 39 | IncRNA zur Phospholipase | genomisch DNA | B73 |
| 40 | IncRNA zur Phosphoinositolphosphatase | Polypeptid (Variante 1) | B73 |
| 41 | IncRNA zur Phosphoinositolphosphatase | Polypeptid (Variante 2) | B73 |

In einem weiteren Aspekt betrifft die vorliegende Erfindung einen Vektor, welcher die erfindungsgemäße Nukleinsäure umfasst. Bei dem Vektor kann es sich um ein Plasmid, ein Cosmid, eine Phage oder einen Expressionsvektor, einen Transformationsvektor, Shuttle-Vektor oder Klonierungsvektor handeln, er kann doppel- oder einzelsträngig, linear oder zirkulär sein oder kann einen prokaryotischen oder eukaryotischen Wirt entweder durch Integration in dessen Genom oder extrachromosomal transformieren. Bevorzugt ist die erfindungsgemäße Nukleinsäure in einem Vektor mit einer oder mehreren regulatorischen Sequenzen, welche die Transkription und optional die Expression in einer prokaryotischen oder eukaryotischen Wirtszelle erlauben, operativ verknüpft. Eine regulatorische Sequenz, vorzugsweise DNA, kann homolog oder heterolog zu der erfindungsgemäßen Nukleinsäure sein. Beispielsweise steht die Nukleinsäure unter der Kontrolle eines geeigneten Promotors oder eines Terminators. Geeignete Promotoren können Promotoren sein, welche konstitutiv induziert werden (Bsp.: 35S-Promoter aus dem "Cauliflower mosaic virus" (Odell et al. 1985), besonders geeignet sind solche Promotoren, welche gewebespezifisch sind (Bsp.: Pollenspezifische Promotoren, Chen et al. (2010), Zhao et al. (2006) oder Twell et al. (1991), oder entwicklungsspezifisch sind (Bsp.: Blühspezifische Promotoren). Geeignete Promotoren können auch synthetische bzw. chimäre Promotoren sein, welche in der Natur nicht vorkommen, aus mehreren Elementen zusammengesetzt sind und einen Minimalpromotor beinhalten sowie stromaufwärts des Minimalpromotors mindestens ein cis-regulatorisches Element aufweisen, welches als Bindungsstelle für spezielle Transkriptionsfaktoren dient. Chimäre Promotoren können den gewünschten Spezifitäten nach konzipiert und werden durch unterschiedliche Faktoren induziert oder reprimiert. Beispiele für solche Promotoren finden sich in Gurr & Rushton (2005) oder Venter (2007). Ein geeigneter Terminator ist beispielsweise der nos-Terminator (Depicker et al., 1982).

Zusätzlich zu den oben beschriebenen Vektoren, stellt die vorliegende Erfindung auch ein Verfahren bereit, das die Einbringung eines beschriebenen Vektors in eine Wirtszelle umfasst. Der Vektor kann beispielsweise durch Konjugation, Mobilisation, biolistische Transformation, Agrobakterium-vermittelte Transformation, Transfektion, Transduktion, Vakuuminfiltration oder Elektroporation eingebracht werden. Solche Verfahren ebenso wie Verfahren zur Präparation von beschriebenen Vektoren sind dem Fachmann geläufig (Sambrook et al. 2001).

In einem weiteren Aspekt betrifft die vorliegende Erfindung eine Wirtszelle, welche die erfindungsgemäße Nukleinsäure oder den Vektor der vorliegenden Erfindung umfasst. Eine Wirtszelle im Sinne der Erfindung kann eine prokaryotische (z.B. bakteriell) oder eukaryotische Zelle (z.B. eine pflanzliche Zelle oder eine Hefezelle) sein. Vorzugsweise ist die Wirtszelle ein Agrobakterium wie *Agrobacterium tumefaciens* oder *Agrobacterium rhizogenes* oder eine Pflanzenzelle, welche die erfindungsgemäße Nukleinsäure oder den Vektor der vorliegenden Erfindung umfassen. Dem Fachmann sind sowohl zahlreiche Methoden wie Konjugation oder Elektroporation bekannt, mit denen er die erfindungsgemäße Nukleinsäure oder den Vektor der vorliegenden Erfindung in ein Agrobakterium einbringen kann, als auch Methoden wie diverse Transformationsverfahren (biolistische Transformation, Agrobakterium-vermittelte Transformation), mit denen er die erfindungsgemäße Nukleinsäure oder den Vektor der vorliegenden Erfindung in eine Pflanzenzelle einbringen kann (Sambrook et al. 2001).

In einem weiteren Aspekt betrifft die vorliegende Erfindung eine transgene Pflanzenzelle, welche die erfindungsgemäße Nukleinsäure als Transgen oder den Vektor der vorliegenden Erfindung umfasst, und eine transgene Pflanze oder einen Teil davon, welche die transgene Pflanzenzelle umfassen. Eine solche transgene Pflanzenzelle oder Pflanze ist beispielsweise eine Pflanzenzelle oder Pflanze, welche mit der erfindungsgemäßen Nukleinsäure oder mit dem Vektor der vorliegenden Erfindung, vorzugsweise stabil, transformiert ist. Eine transgene Pflanze der vorliegenden Erfindung ist vorzugsweise zur Verwendung als Haploideninduktor geeignet. In einer bevorzugten Ausgestaltung der transgenen Pflanze ist die Nukleinsäure mit einer oder mehreren regulatorischen Sequenzen, welche die Transkription und optional die Expression in der Pflanzenzelle erlauben, operativ verknüpft. Eine regulatorische Sequenz, vorzugsweise DNA, kann homolog oder heterolog zu der erfindungsgemäßen Nukleinsäure sein. Das Gesamtkonstrukt aus der erfindungsgemäßen Nukleinsäure und der/den regulatorischen Sequenz(en) können dann das Transgen darstellen. Ein Teil einer Pflanze kann ein befruchteter oder unbefruchteter Samen, ein Embryo, ein Pollen, ein Gewebe, ein Organ oder eine pflanzliche Zelle sein, wobei der befruchtete oder unbefruchtete Samen, der Embryo oder der Pollen auf der transgenen Pflanze erzeugt werden und in deren Genom die erfindungsgemäße Nukleinsäure als Transgen oder der Vektor integriert ist. Ebenso schließt die vorliegende Erfindung auch ein Nachkomme der transgenen Pflanze ein, in dessen Genom die erfindungsgemäße Nukleinsäure als Transgen oder der Vektor integriert ist und der zur Verwendung als Haploideninduktor geeignet ist.

In einem anderen Aspekt betrifft die vorliegende Erfindung ein Protein oder ein Polypeptid, welches durch eine erfindungsgemäße Nukleinsäure kodiert wird. Bevorzugt ist das Protein oder Polypeptid geeignet, in einer Pflanze die Eigenschaft eines Haploideninduktors zu vermitteln oder die Induktionsleistung eines Haploideninduktors zu erhöhen. Besonders bevorzugt wird das Protein oder Polypeptid durch die induktionsinduzierende Nukleinsäure kodiert. Vorzugsweise umfasst ein Protein oder ein Polypeptid der vorliegenden Erfindung eine Aminosäuresequenz ausgewählt aus SEQ ID NOs: 23.

In einem weiteren Aspekt beschreibt die vorliegende Erfindung ein Verfahren zur Herstellung einer Pflanze, welche zur Verwendung als Haploideninduktor geeignet ist. Das Verfahren kann die folgenden Schritte umfassen:
A) Mutagenisieren von Pflanzenzellen und anschließendem Regenerieren von Pflanzen aus den mutagenisierten Pflanzenzellen oder Mutagenisieren von Pflanzen, und
B) Identifizieren einer Pflanze aus A), welche in einer endogenen DNA-Sequenz, welche mit der erfindungsgemäßen Nukleinsäure identisch ist, oder in einer regulatorischen Sequenz der endogenen DNA-Sequenz wie beispielsweise einem Promoter, Enhancer, Terminator oder Intron mindestens eine Mutation aufweist, die eine Änderung der Transkriptions- oder Expressionsrate der endogenen DNA-Sequenz in der identifizierten Pflanze im Vergleich mit einer nicht-mutagenisierten Wildtyp-Pflanze oder eine Änderung der Aktivität oder Stabilität eines durch die endogene DNA-Sequenz kodierten Proteins oder Polypeptids in der identifizierten Pflanze im Vergleich mit einer nicht-mutagenisierten Wildtyp-Pflanze bewirkt, wobei die mindestens eine Mutation verursacht, dass in der identifizierten Pflanze die Eigenschaft eines Haploideninduktors vermittelt wird oder die Induktionsleistung eines Haploideninduktors erhöht wird. Vorzugsweise zeigt sich die Änderung der Transkriptions- oder Expressionsrate oder die Änderung der Aktivität oder Stabilität mindestens in einem Pollen der identifizierten Pflanze oder in einem Gewebe eines Pollens der identifizierten Pflanze.

Bevorzugt hat die endogene DNA-Sequenz aus Schritt B) oder eine von der endogenen DNA-Sequenz kodierte RNA oder ein von der endogenen DNA-Sequenz kodiertes Protein oder Polypeptid Einfluss auf das Pollenschlauch-Wachstum in einer Pflanze, auf den Energiestoffwechsel eines Pollens einer Pflanze und/oder auf die Aktivität des Centromers vorzugsweise in einer generativen Zelle, welche sich beispielsweise zu einem Pollen entwickelt.

Besonders bevorzugt kodiert die endogene DNA-Sequenz aus Schritt B) des Verfahrens zur Herstellung einer Pflanze, welche zur Verwendung als Haploideninduktor geeignet ist, für ein Enzym der Klasse der Phospoholipasen, insbesondere der Phospholipase A2 oder der Patatin-Phospholipase, wobei die Transkriptions- oder Expressionsrate bzw. die Aktivität oder Stabilität vorzugsweise dahingehend geändert ist, dass sie erhöht ist.

Eine Mutation meint eine Modifikation auf DNA-Ebene, also eine Veränderung der Genetik und/oder der Epigenetik. Beispielsweise kann eine Veränderung der Genetik der Austausch von mindestens einer Nukleobase in der endogenen DNA-Sequenz oder in einer regulatorischen Sequenz der endogenen DNA-Sequenz sein. Findet ein solcher Nukleobasen-Austausch z.B. in einem Promotor statt, kann dies zu einer veränderten Aktivität des Promotors führen, da beispielsweise cis-regulatorische Elemente derart modifiziert werden, dass die Affinität eines Transkriptionsfaktors zu dem mutierten cis-regulatorische Elemente im Vergleich zum Wildtyp-Promotor verändert ist, sodass die Aktivität des Promotors mit dem mutierten cis-regulatorische Elemente erhöht oder erniedrigt ist, je nachdem ob es sich bei dem Transkriptionsfaktor um einen Repressor oder Induktor handelt oder ob die Affinität des Transkriptionsfaktors zu dem mutierten cis-regulatorische Elemente verstärkt oder abgeschwächt wird. Findet ein solcher Nukleobase-Austausch z.B. in einer kodierenden Region der endogenen DNA-Sequenz statt, kann dies zu einem Aminosäureaustausch im kodierten Protein führen, was eine Veränderung der Aktivität oder Stabilität des Proteins im Vergleich zum Wildtyp-Protein bewirken kann. Ein weiteres Beispiel für eine Veränderung der Genetik ist die Deletion von Nukleotiden in der regulatorische Sequenz und/oder der endogenen DNA-Sequenz sowie die Addition von Nukleotiden in der regulatorischen Sequenz und/oder der endogenen DNA-Sequenz. Ein Beispiel zur Regulation von Genen durch Insertion von Nukleotiden durch Transposonmutagenese in Mais zeigt Das & Martienssen (1995). Eine Veränderung der Epigenetik kann beispielsweise durch ein verändertes Methylierungsmuster der DNA erfolgen.

Dem Fachmann ist bekannt, wie eine Mutation im Sinne der Erfindung durch den Prozess einer Mutagenisierung im Schritt A) des Verfahrens zur Herstellung einer Pflanze, welche zur Verwendung als Haploideninduktor geeignet ist, erreicht werden kann. Die Mutagenisierung schließt hierbei sowohl die konventionelle Mutagenese als auch die ortspezifische Mutagenese beziehungsweise das 'Genome Editing' ein. Bei der konventionellen Mutagenese wird die Modifikation auf DNA-Ebene nicht gezielt herbeigeführt. Die Pflanzenzelle oder die Pflanze wird mutagenen Bedingungen wie z.B. TILLING durch UV-Licht-Bestrahlung oder den Einsatz chemischer Stoffe ausgesetzt (Till et al., 2004). Eine weitere Methode der Zufallsmutagenese ist die Mutagenese mit Hilfe eines Transposons. Eine umfangreiche Mutantesammlung steht über das UniformMU-Projekt frei zur Verfügung. Die Sammlung und die Methode sind beschrieben in McCarty et al. (2005). Die ortspezifische Mutagenese ermöglicht die Einbringung von Modifikation auf DNA-Ebene zielgerichtet an vordefinierten Stellen der DNA. Hierfür können beispielsweise, TALENS (WO 2010/079430, WO 2011/072246), Meganukleasen (Silva et al., 2011), Homing-Endonukleasen (Chevalier 2002), Zinkfingernukleasen (Lloyd et al., 2005) oder ein CRISPR/Cas-System (Gaj et al., 2013) verwendet werden.

Das Identifizieren einer Pflanze im Schritt B) kann beispielsweise mit Hilfe von molekularen Markern oder Sonden erfolgen. DNA-Sonden sind beispielsweise Primer oder Primerpaare, welche in einer PCR-Reaktion eingesetzt werden können. Beispielsweise können Tillingmutanten durch Sequenzierung des Zielgens in einer Tilling-Population oder weitere Methoden, die Fehlpaarungen in der DNA nachweisen, wie z.B. Schmelzpunktanalysen oder Einsatz fehlpaarungsspezifischer Nucleasen nachgewiesen beziheungsweise identifiziert werden. Vorliegende Erfindung schließt hierfür einsetzbare Primer/Primerpaare ebenfalls mit ein, wie z.B. Primer für Phospholipase, Phosphoglyceratmutase, Methyltransferase und IncRNA zu Phospholipase. Ferner können Mutanten, erzeugt mittels Transposons, durch Einsatz von Transposon-spezfischem Primern und Zielgen-spezifischem Primern in der PCR über die gesamte Population und anschließende Sequenzierung von PCR-Produkten nachgewiesen werden. Auch solche Primer sind von der vorliegenden Erfindung mit erfasst. Änderung der Expressionsrate können beispielsweise mit RT-PCR in Pollen bestimmt werden, die Änderung der Stabilität beispielsweise durch Untersuchung von Ubiquitin-Bindestellen und Vorhersagen über Änderungen der Teritärstruktur. Weiterhin sind auch rekombinante Expression der Wildtyp-Proteine und der enstprechenden mutanten Proteine und nachfolgende biochemische Aktivitätstests geeignet. Dem Fachmann sind aus dem Stand der Technik weitere Mittel und Verfahren bekannt, welche er zum Identifizieren einer Pflanze im Schritt B) verwenden kann. Die vorliegende Erfindung betrifft auch molekulare Marker, welche die Anwesenheit oder Abwesenheit einer Mutation in der endogenen DNA-Sequenz oder in einer regulatorischen Sequenz der endogenen DNA-Sequenz nachweisen. Solche Marker basieren beispielsweise auf einem SNP und sind spezifisch für die Mutation (Beispiele: KASPar oder TaqMan Marker).

Die vorliegende Erfindung betrifft weiterhin auch eine Pflanze, welche mit dem vorstehenden Verfahren herstellbar ist oder hergestellt ist, oder ein Teil dieser Pflanze, wobei ein Teil einer Pflanze ein befruchteter oder unbefruchteter Samen, ein Embryo, ein Pollen, ein Gewebe, ein Organ oder eine pflanzliche Zelle sein kann, wobei der befruchtete oder unbefruchtete Samen, der Embryo oder der Pollen auf der transgenen Pflanze erzeugt werden und in deren Genom die mindestens eine Mutation vorhanden ist. Ebenso schließt die vorliegende Erfindung auch ein Nachkomme der Pflanze ein, welcher die mindestens eine Mutation aufweist und zur Verwendung als Haploideninduktor geeignet ist.

Ferner betrifft die vorliegende Erfindung auch ein Verfahren zur Isolierung einer Nukleinsäure, die in einer Pflanze die Eigenschaft eines Haploideninduktors vermittelt oder die Induktionsleistung eines Haploideninduktors erhöht, umfassend die folgenden Schritte:
A) Herstellung einer Pflanze nach vorstehend beschriebenen Verfahren oder Bereitstellung einer Pflanze, welche mit vorstehend beschriebenen Verfahren herstellbar ist oder hergestellt ist, und B) Isolieren einer Nukleinsäure, welche die endogene DNA-Sequenz mit der mindestens einen Mutation umfasst, aus dem Genom der Pflanze aus A). Das Isolieren der Nukleinsäure in Schritt B) kann durch CTAB-Extraktion oder über DNA-bindende Säulen erfolgen, der Nachweis der Mutation über Sequenzierung oder molekulare Marker, wie SNP basierte KASPar oder TaqMan Marker, oder bei Insertions- oder Deletionsmutanten über Marker auf der Basis von Längenpolymorphismen.

Die vorliegende Erfindung schließt auch eine Nukleinsäure ein, welche durch das vorstehend beschriebenen Verfahren zur Isolierung erhalten wurde oder durch das vorstehend beschriebenen Verfahren zur Isolierung erhältlich ist, sowie einen Vektor, welcher die isolierte Nukleinsäure umfasst.

In einem anderen Aspekt betrifft die vorliegende Erfindung auch ein Verfahren zur Herstellung einer transgenen Pflanze, welche zur Verwendung als Haploideninduktor geeignet ist. Das Verfahren kann die folgenden Schritte umfassen:
A) Bereitstellen der oben beschriebenen Nukleinsäure, welche nach Transkription oder nach Expression in einer Pflanze geeignet ist, die Eigenschaft eines Haploideninduktors zu vermitteln oder die Induktionsleistung eines Haploideninduktors zu erhöhen, oder Bereitstellen der oben beschriebenen isolierten Nukleinsäure, welche die endogene DNA-Sequenz mit der mindestens einen Mutation umfasst, oder Bereitstellen von einem der oben beschriebenen Vektoren,
B) Transformieren, vorzugsweise stabil Transformieren, von Pflanzenzellen durch Einbringen der Nukleinsäure oder des Vektors aus A),
C) Regenerieren transgener Pflanzen aus den transformierten Pflanzenzellen aus B), und
D) Identifizieren einer transgenen Pflanze aus C), in welcher durch ein geändertes Expressionsmuster, vorzugsweise im Pollen der identifizierten Pflanze oder in einem Gewebe eines Pollens der identifizierten Pflanze, die Eigenschaft eines Haploideninduktors vermittelt wird oder die Induktionsleistung eines Haploideninduktors erhöht wird. Das Verfahren zur Herstellung einer transgenen Pflanze, welche zur Verwendung als Haploideninduktor geeignet ist, schließt auch die Bereitstellung von zwei oder mehr der oben beschriebenen Nukleinsäure, wahlweise auch unterschiedliche Ausgestaltungen der erfindungsgemäßen Nukleinsäure und optional in einem oder mehreren Vektoren, und das Transformieren von Pflanzenzellen durch Einbringen der zwei oder mehr Nukleinsäuren ein. Alternativ oder ergänzend können neben der erfindungsgemäßen Nukleinsäure auch eine oder mehrere weitere Nukleinsäuren, welche bekanntermaßen einsetzbar sind zur Erzeugung eines Haploideninduktors (z.B. manipuliertes cenh3-Gen (Ravi & Chan, 2010) bereitgestellt und transformiert werden.

Bevorzugt ist das Expressionsmuster dahingehend geändert, dass
(I) die Transkriptions- oder Expressionsrate der eingebrachten induktionsfördernden Nukleinsäure in der identifizierten Pflanze im Vergleich zu einer Wildtyp-Pflanze, welche beispielsweise aus einer isogenen, nicht-transformierten Pflanzenzelle regeneriert wurde, erhöht ist, Ein Nachweis der Transkriptionsrate kann beispielsweise über qRT-PCR erfolgen. Eine geänderte Proteinstabilität kann zum Beispiel über Western Blot bestimmt werden.

Die vorliegende Erfindung betrifft weiterhin auch eine transgene Pflanze, welche mit diesem Verfahren herstellbar ist oder hergestellt ist, oder ein Teil dieser Pflanze, wobei ein Teil einer Pflanze ein befruchteter oder unbefruchteter Samen, ein Embryo, ein Pollen, ein Gewebe, ein Organ oder eine pflanzliche Zelle sein kann, wobei der befruchtete oder unbefruchtete Samen, der Embryo oder der Pollen auf der transgenen Pflanze erzeugt werden und in deren Genom die erfindungsgemäße Nukleinsäure als Transgen oder der Vektor integriert ist. Ebenso schließt die vorliegende Erfindung auch ein Nachkomme der transgenen Pflanze ein, welcher die eingebrachte Nukleinsäure als Transgen aufweist und zur Verwendung als Haploideninduktor geeignet ist.

In einem anderen Aspekt betrifft die vorliegende Offenbarung ein Verfahren zur Herstellung einer haploiden Pflanze, welches die folgenden Schritte umfasst:
A) Kreuzen einer nicht-transgenen oder transgenen Pflanze der vorliegenden Erfindung, welche zur Verwendung als Haploideninduktor geeignet ist, mit einer Pflanze derselben Gattung, vorzugsweise derselben Spezies,
B) Selektieren eines befruchtete haploiden Samens oder Embryos, und
C) Erzeugen einer haploiden Pflanze aus dem Samen oder Embryo aus B).

Bevorzugt wird die Pflanze, welche zur Verwendung als Haploideninduktor geeignet ist, als Pollenelter eingesetzt und mit einem Saatelter derselben Gattung, vorzugsweise derselben Spezies gekreuzt. Es kann auch die Pflanze, welche zur Verwendung als Haploideninduktor geeignet ist, als Saatelter eingesetzt werden und mit einem Pollenelter derselben Gattung, vorzugsweise derselben Spezies gekreuzt werden. Beide Kreuzungspartner in Schritt A), also Saatelter und Pollenelter, können auch dasselbe Individuum sein. Dann stellt der Kreuzungsschritt eine Selbstung dar.

Das Selektieren des haploiden befruchteten Samens oder Embryos kann einen Schritt des Nachweises der Haploidie und des Separierens des haploiden befruchteten Samens oder Embryos von polyploiden befruchteten Samen oder Embryos umfassen. Der Nachweis der Haploidie eines befruchteten Samens oder Embryos kann phänotypisch oder genotypisch erfolgen, indem beispielsweise der Induktor mit einem embryo-spezifischen dominanten Marker versehen wird, der in allen diploiden Nachkommen, aber nicht in den induzierten haploiden Nachkommen sichtbar wird. Des Weiteren kann der Ploidie-Status über Flowcytometrie bestimmt werden. Außerdem gibt ein vollständig homozygotes Muster von molekularen Markern einen Hinweis auf haploide Pflanzen. Das Separieren kann automatisiert beispielsweise auf Basis von Daten des Nachweises der Haploidie erfolgen.

Die vorliegende Erfindung betrifft weiterhin auch einen haploiden befruchteten Samen oder Embryo, welcher beim Kreuzen in Schritt A) des Verfahrens zur Herstellung einer haploiden Pflanze entsteht sowie eine haploide Pflanze, welche mit diesem Verfahren herstellbar ist oder hergestellt ist, oder ein Teil dieser Pflanze, wobei ein Teil einer Pflanze ein Samen, ein Embryo, ein Gewebe, ein Organ oder eine pflanzliche Zelle sein kann. Ebenso schließt die vorliegende Erfindung auch ein Nachkomme der Pflanze ein. Weiterhin erfasst die vorliegende Erfindung auch eine doppelhaploide (diploide) Pflanze oder ein Teil davon, wobei die doppelhaploide (diploide) Pflanze oder der Teil davon durch Chromosomendoppelung der haploiden Pflanze oder des Teils davon erzeugt wurde.

Doppelsträngige RNA zur Herstellung des erfindungsgemäßen Mittels kann *in vitro* mittels dem Fachmann bekannter Methoden produziert werden. Beispielsweise kann die Synthese der doppelsträngigen RNA synthetisch erfolgen, wobei die RNA *in vitro* direkt gebildet wird. Die doppelsträngige RNA kann auch ausgehend von einer doppelsträngigen DNA über die Bildung eines mRNA-Transkriptes, welches dann z.B. eine hairpin-Struktur ausbildet, synthetisiert wird. Das Mittel kann als Auslöser für eine Haploideninduktion in einer Pflanze verwendet werden. Beispielsweise kann das Mittel durch Aufsprühen in Form eines Sprays oder weiteren dem Fachmann geläufigen Wegen der äußeren Applikation auf das Pflanzengewebe oder durch Aufsprühen oder Mischen mit anderen Additiven vor oder nach der Blüte der Pflanzen eingesetzt werden. Additive können beispielsweise Benetzungsmittel, Trägersubstanzen oder RNA-Stabilisatoren wie z.B. Liposomen sein.

Die Erfinder haben überraschenderweise festgestellt, dass gerade Gene oder Genprodukte, welche Einfluss auf das Pollenschlauch-Wachstum, auf den Energiestoffwechsel eines Pollens und/oder auf die Aktivität des Centromers vorzugsweise in einer generativen Zelle, welche sich beispielsweise zu einem Pollen entwickelt, besonders geeignet sind einen Nicht-Haploideninduktor in einen Haploideninduktor umzuwandeln. Hierfür konnten mehrere Genfamilien/Proteinfamilien identifiziert werden, welche von wesentlicher Bedeutung sind. Ihre Verwendung zur Erzeugung von Haploideninduktoren ist im Stand der Technik bisher weder beschreiben noch nahegelegt. Da die Entstehung von Pollen und auch der Befruchtungsprozess (einschließlich des Auswachsens des Pollenschlauchs) allgemeingültigen Prinzipien bei mono- und dikotyledonen Pflanzen folgt, erhält der Fachmann mit der technischen Lehre der vorliegenden Erfindung die Möglichkeit Haploideninduktoren auch für Kulturpflanzen zu entwickeln, für die bisher ein effizientes System weder der *in vivo-*Haploideninduktion noch anderer Zellkultur-basierter Verfahren zur Erstellung doppelhaploider Pflanzen noch nicht existieren. Hierfür kann er anhand der genetischen Informationen, welche er durch die vorliegende Erfindung erhält, durch Routinetätigkeit Homologe, Orthologe oder Analoge der beschreibenen Genprodukte auffinden und wie hier beschrieben manipulieren. Die technische Lehre der vorliegenden Erfindung ist aber auch geeignet, die bereits bestehenden Induktoren hinsichtlich Ihrer Effizienz (d.h. Haploideninduktionsrate) noch zu verbessern und so erst wirtschaftlich anwendbar zu machen. Weiterhin kann ein Fachmann diese technische Lehre auch mit weiteren bekannten Mechanismen der Haploideninduktion wie eine Manipulation des CENH3-Proteins (Ravi & Chan, 2010) kombinieren und so die Effizienz weiter steigern.

Einige der in dieser Anmeldung verwendeten Begriffe werden nachfolgend näher erläutert: "B73" ist eine Maisinzuchtlinie, die als Model-Genotyp in der Maisgenetik eingesetzt wird und zur Erstellung der ersten Maisreferenzsequenz eingesetzt wurde.

"Die Eigenschaft eines Haploideninduktors vermitteln" oder das "Vermitteln der Eigenschaft eines Haploideninduktors" oder ein vergleichbarer Ausdruck bedeutet, dass eine Pflanze durch Verwendung einer erfindungsgemäßen Nukleinsäure in die Lage versetzt wird, befruchtete Samen oder Embryos, welche einen einfachen (haploiden) Chromosomensatz aufweisen, aus einer Kreuzung mit einer Pflanze derselben Gattung, vorzugsweise derselben Spezies, welche nicht die Eigenschaft eines Haploideninduktors aufweist, hervorzubringen. Die Eigenschaft eines Haploideninduktors, angegeben als absolute Haploiden-Induktionsrate, meint, dass mindestens 0,1%, 0,2%, 0,3%, 0,4%, 0,5%, 0,6%, 0,7%, 0,8%, 0,9% oder 1%, bevorzugt mindestens 1,5%, 2%, 2,5%, 3%, 3,5%, 4%, 4,5% oder 5%, besonders bevorzugt mindestens 6%, 7%, 8%, 9%, 10%, 11%, 12%, 13%, 14% oder 15%, oder ganz besonders bevorzugt mindestens 20%, 25%, 30%, 35%, 40%, 45% oder 50% der befruchteten Samen oder Embryos einen haploiden Chromosomensatz aufweisen.

"Erhöhung der Expressionsrate" oder "erhöhte Expressionsrate" oder "Aktivierung der Expression" oder ein vergleichbarer Ausdruck bedeutet eine Erhöhung der Expressionsrate einer Nukleotidsequenz um mehr als 10%, 15%, 20%, 25% oder 30%, bevorzugt um mehr als 40%, 50%, 60%, 70%, 80%, 90% oder 100%, besonders bevorzugt um mehr als 150%, 200%, 250%, 300%, 500% oder 1000% im Vergleich zu der angegebenen Referenz. Vorzugsweise führt die Erhöhung der Expressionsrate zu einer Änderung des Phänotyps einer Pflanze, in welcher die Expressionsrate erhöht ist. Ein geänderter Phänotyp kann die Vermittlung der Eigenschaft eines Haploideninduktors oder die Erhöhung der Induktionsleistung eines Haploideninduktors sein.

"Erhöhung der Transkriptionsrate" oder "erhöhte Transkriptionsrate" oder ein vergleichbarer Ausdruck bedeutet eine Erhöhung der Transkriptionsrate einer Nukleotidsequenz um mehr als 10%, 15%, 20%, 25% oder 30%, bevorzugt um mehr als 40%, 50%, 60%, 70%, 80%, 90% oder 100%, besonders bevorzugt um mehr als 150%, 200%, 250%, 300%, 500% oder 1000% im Vergleich zu der angegebenen Referenz. Vorzugsweise führt die Erhöhung der Transkriptionsrate zu einer Änderung des Phänotyps einer Pflanze, in welcher die Transkriptionsrate erhöht ist. Ein geänderter Phänotyp kann die Vermittlung der Eigenschaft eines Haploideninduktors oder die Erhöhung der Induktionsleistung eines Haploideninduktors sein.

Ein "funktionelles Fragment" einer Nukleotidsequenz meint einen Abschnitt einer Nukleotidsequenz, welcher die identische oder eine vergleichbare Funktionalität wie die Gesamtnukleotidsequenz, aus welcher das funktionelle Fragment stammt, aufweist. Als solches kann das funktionelle Fragment eine Nukleotidsequenz besitzen, welche über eine Länge von mindestens 50%, 55%, 60%, 65%, 70%, 75%, 80%, 85%, 90%, 92%, 94% 96%, 97%, 98% oder 99% identisch oder homolog mit der Gesamtnukleotidsequenz ist. Weiterhin kann ein "funktionelles Fragment" einer Nukleotidsequenz auch einen Abschnitt einer Nukleotidsequenz meinen, welcher die Funktionalität der Gesamtnukleotidsequenz beispielsweise im Zuge von posttranskriptionalem oder transkriptionellem Gene Silencing verändert. Als solches kann das funktionelle Fragment einer Nukleotidsequenz mindestens 14, 15, 16, 17, 18, 19, 20, 21, 22, 23, 24 oder 25, bevorzugt mindestens 30, 35, 40, 45, 50, 60, 70, 80, 90, 100, 120 oder 140, besonders bevorzugt mindestens 160, 180, 200, 250, 300, 350, 400, 450, 500, 600, 700, 800, 900 oder 1000 aufeinanderfolgende Nukleotide der Gesamtnukleotidsequenz umfassen.

Ein "funktioneller Teil" eines Proteins meint einen Abschnitt eines Proteins bzw. eine Anschnitt der Aminosäuresequenz, die das Protein kodiert, wobei der Abschnitt die identische oder eine vergleichbare Funktionalität wie das Gesamtprotein in einer pflanzlichen Zelle ausüben kann. Ein funktioneller Teil eines Proteins weist über eine Länge von mindestens 50%, 55%, 60%, 65%, 70%, 75%, 80%, 85%, 90%, 92%, 94% 96%, 97%, 98% oder 99% eine identische oder unter Brücksichtigung von konservativen und semikonservativen Aminosäureaustauschen ähnliche Aminosäuresequenz auf wie das Protein, aus welchem der funtionelle Teil stammt "Haploideninduktor" meint auch einen in vivo-Haploideninduktor.

Der Begriff "heterolog" bedeutet, dass das eingebrachte Polynukleotid beispielsweise von einer Zelle oder einem Organismus mit einem anderen genetischen Hintergrund derselben Spezies oder einer anderen Spezies stammt, oder homolog ist zu der prokaryotischen oder eukaryotischen Wirtszelle, dann aber in einer unterschiedlichen genetischen Umgebung lokalisiert ist und sich so von einem eventuell natürlicherweise vorhandenen korrespondierenden Polynukleotid unterscheidet. Ein heterologes Polynukleotid kann zusätzlich zu einem entsprechenden endogenen Gen vorhanden sein.

Im Sinne der Erfindung wird unter einem "Homolog" ein Protein gleichen phylogenetischen Ursprungs verstanden, unter einem "Analog" ein Protein verstanden, welches die gleiche Funktion ausübt, jedoch einen anderen phylogenetischen Ursprung hat, und unter einem "Ortholog" ein Protein aus einer anderen Spezies verstanden, das die gleiche Funktion ausübt.

Unter "Hybridisieren" oder "Hybridisierung" wird ein Vorgang verstanden, bei dem sich ein einzelsträngiges Nukleinsäuremolekül an einen weitestgehend komplementären Nukleinsäurestrang anlagert, d.h. Basenpaarungen eingeht. Standardverfahren zur Hybridisierung sind beispielsweise in Sambrook et al. 2001 beschrieben. Vorzugsweise wird darunter verstanden, dass mindestens 60%, weiter bevorzugt mindestens 65%, 70%, 75%, 80% oder 85%, besonders bevorzugt 90%, 91%, 92%, 93%, 94%, 95%, 96%, 97%, 98% oder 99% der Basen des Nukleinsäuremoleküls eine Basenpaarung mit dem weitestgehend komplementären Nukleinsäurestrang eingehen. Die Möglichkeit einer solchen Anlagerung hängt von der Stringenz der Hybridisierungsbedingungen ab. Der Begriff "Stringenz" bezieht sich auf die Hybridisierungsbedingungen. Hohe Stringenz ist dann gegeben, wenn eine Basenpaarung erschwert ist, niedrige Stringenz, wenn eine Basenpaarung erleichtert ist. Die Stringenz der Hybridisierungsbedingungen hängt beispielsweise von der Salzkonzentration bzw. lonenstärke und der Temperatur ab. Generell kann die Stringenz durch eine Erhöhung der Temperatur und/oder eine Erniedrigung des Salzgehaltes erhöht werden. Unter "stringenten Hybridisierungsbedingungen" sind solche Bedingungen zu verstehen, bei denen eine Hybridisierung überwiegend nur zwischen homologen Nukleinsäuremolekülen stattfindet. Der Begriff "Hybridisierungsbedingungen" bezieht sich dabei nicht nur auf die bei der eigentlichen Anlagerung der Nukleinsäuren herrschenden Bedingungen, sondern auch auf die bei den anschließenden Waschschritten herrschenden Bedingungen. Stringente Hybridisierungsbedingungen sind beispielsweise Bedingungen, unter denen überwiegend nur solche Nukleinsäuremoleküle hybridisieren, die mindestens 70%, vorzugsweise mindestens 75%, mindestens 80%, mindestens 85%, mindestens 90% oder mindestens 95% Sequenzidentität aufweisen. Stringente Hybridisierungsbedingungen sind beispielsweise: Hybridisieren in 4 × SSC bei 65 °C und anschließendes mehrfaches Waschen in 0,1 × SSC bei 65 °C für insgesamt etwa 1 Stunde. Der hier verwendete Begriff "stringente Hybridisierungsbedingungen" kann auch bedeuten: Hybridisieren bei 68 °C in 0,25 M Natriumphosphat, pH 7,2, 7 % SDS, 1 mM EDTA und 1 % BSA für 16 Stunden und anschließendes zweimaliges Waschen mit 2 × SSC und 0,1 % SDS bei 68 °C. Bevorzugt findet eine Hybridisierung unter stringenten Bedingungen statt.

"Die Induktionsleistung eines Haploideninduktors erhöhen" oder "die Erhöhung der Induktionsleistung eines Haploideninduktors" meint, dass die Haploiden-Induktionrate einer Pflanze, welche die Eigenschaft eines Haploideninduktors aufweist, erhöht wird. Somit kann die Zahl der befruchteten Samen, welche einen haploiden Chromosomensatz aufweisen und aus einer Kreuzung des Haploideninduktors mit einer Pflanze derselben Gattung, vorzugsweise derselben Spezies, welche nicht die Eigenschaft eines Haploideninduktors aufweist, hervorgegangen sind, um mindestens 0,1%, 0,2%, 0,3%, 0,4%, 0,5%, 0,6%, 0,7%, 0,8%, 0,9% oder 1%, bevorzugt mindestens 1,5%, 2%, 2,5%, 3%, 3,5%, 4%, 4,5% oder 5%, und besonders bevorzugt mindestens 6%, 7%, 8%, 9%, 10%, 15%, 20%, 30% oder 50% höher sind als die Zahl der haploiden befruchteten Samen, welche ohne die Verwendung der Nukleinsäure im Sinne der vorliegenden Erfindung erzielt wird, d.h. die Haploiden-Induktionsrate kann um mindestens 0,1%, 0,2%, 0,3%, 0,4%, 0,5%, 0,6%, 0,7%, 0,8%, 0,9% oder 1%, bevorzugt mindestens 1,5%, 2%, 2,5%, 3%, 3,5%, 4%, 4,5% oder 5%, und besonders bevorzugt mindestens 6%, 7%, 8%, 9%, 10%, 15%, 20%, 30% oder 50% relativ zu der zuvor erreichten Haploiden-Induktionsrate gesteigert werden.

"Operativ verknüpft" meint verbunden in einem gemeinsamen Nukleinsäuremolekül, in einer Weise, dass die verbundenen Elemente derart zueinander positioniert und orientiert sind, dass eine Transkription des Nukleinsäuremoleküls stattfinden kann. Eine DNA, welche operativ mit einem Promotor verknüpft ist, steht unter der transkriptionellen Kontrolle dieses Promotors.

Pflanzliche "Organe" meinen beispielsweise Blätter, Sprossachse, Stamm, Wurzeln, vegetative Knospen, Meristeme, Embryos, Antheren, Ovula oder Früchte. Pflanzliche "Teile" meinen einen Zusammenschluss mehrerer Organe, z.B. eine Blüte oder ein Samen, oder einen Teil eines Organs, z.B. einen Querschnitt aus der Sprossachse. Pflanzliche "Gewebe" sind zum Beispiel Kallusgewebe, Speichergewebe, meristematische Gewebe, Blattgewebe, Sprossgewebe, Wurzelgewebe, Pflanzentumorgewebe oder reproduktives Gewebe. Unter pflanzlichen "Zellen" sind beispielsweise isolierte Zellen mit einer Zellwand oder Aggregate davon oder Protoplasten zu verstehen.

Eine "Pflanze" im Sinne der Erfindung kann, sofern nicht anders angegeben von jeder Spezies aus den dikotyledonen, monokotyledonen und gymnospermen Pflanzen sein. Hierzu zählen beispielhaft *Hordeum vulgare*, *Sorghum bicolor*, *Secale cereale*, Triticale, *Saccharum officinarium*, *Zea mays*, *Setaria italic*, *Oryza sativa*, *Oryza minuta*, *Oryza australiensis*, *Oryza alta*, *Triticum aestivum*, *Triticum durum*, *Hordeum bulbosum*, *Brachypodium distachyon*, *Hordeum marinum*, *Aegilops tauschii*, *Beta vulgaris*, *Helianthus annuus*, *Daucus glochidiatus*, *Daucus pusillus*, *Daucus muricatus*, *Daucus carota*, *Eucalyptus grandis*, *Erythranthe guttata*, *Genlisea aurea*, *Gossypium sp.*, *Musa sp.*, *Avena sp., Nicotiana sylvestris*, *Nicotiana tabacum*, *Nicotiana tomentosiformis*, *Solanum lycopersicum*, *Solanum tuberosum*, Coffea *canephora*, *Vitis vinifera*, *Cucumis sativus*, *Morus notabilis*, *Arabidopsis thaliana*, *Arabidopsis lyrata*, *Arabidopsis arenosa*, *Crucihimalaya himalaica*, *Crucihimalaya wallichii*, *Cardamine flexuosa*, *Lepidium virginicum*, *Capsella bursa-pastoris*, *Olmarabidopsis pumila, Arabis hirsuta*, *Brassica napus*, *Brassica oleracea*, *Brassica rapa*, *Brassica juncacea*, *Brassica nigra*, *Raphanus sativus*, *Eruca vesicaria sativa*, *Citrus sinensis*, *Jatropha curcas*, *Glycine max* und *Populus trichocarpa.* Eine erfindungsgemäße Pflanze ist vorzugsweise eine Pflanze der Gattung Zea, insbesondere der Spezies Zea mays, oder Sorghum.

"Reduzierung der Expressionsrate" oder "Reduktion der Expressionsrate" oder "Unterdrückung der Expression" "reduzierte Expressionsrate" oder ein vergleichbarer Ausdruck bedeutet eine Reduzierung der Expressionsrate einer Nukleotidsequenz um mehr als 10%, 15%, 20%, 25% oder 30%, bevorzugt um mehr als 40%, 45%, 50%, 55%, 60%, oder 65%, besonders bevorzugt um mehr als 70%, 75%, 80%, 85%, 90%, 92%, 94%, 96% oder 98% im Vergleich zu der angegebenen Referenz. Es kann aber auch bedeuten, dass die Expressionsrate einer Nukleotidsequenz um 100% reduziert wird. Vorzugsweise führt die Reduzierung der Expressionsrate zu einer Änderung des Phänotyps einer Pflanze, in welcher die Expressionsrate reduziert ist. Ein geänderter Phänotyp kann die Vermittlung der Eigenschaft eines Haploideninduktors oder die Erhöhung der Induktionsleistung eines Haploideninduktors sein.

"Reduzierung der Transkriptionsrate" oder "reduzierte Transkriptionsrate" oder ein vergleichbarer Ausdruck bedeutet eine Reduzierung der Transkriptionsrate einer Nukleotidsequenz um mehr als 10%, 15%, 20%, 25% oder 30%, bevorzugt um mehr als 40%, 45%, 50%, 55%, 60%, oder 65%, besonders bevorzugt um mehr als 70%, 75%, 80%, 85%, 90%, 92%, 94%, 96% oder 98% im Vergleich zu der angegebenen Referenz. Es kann aber auch bedeuten, dass die Expressionsrate einer Nukleotidsequenz um 100% reduziert wird. Vorzugsweise führt die Reduzierung der Transkriptionsrate zu einer Änderung des Phänotyps einer Pflanze, in welcher die Transkriptionsrate reduziert ist. Ein geänderter Phänotyp kann die Vermittlung der Eigenschaft eines Haploideninduktors oder die Erhöhung der Induktionsleistung eines Haploideninduktors sein.

Im Zusammenhang mit der vorliegenden Erfindung betrifft der Begriff "regulatorische Sequenz" eine Nukleotidsequenz, welche die Spezifität und/oder die Expressionsstärke beeinflusst, zum Beispiel indem die regulatorische Sequenz eine bestimmte Gewebespezifität vermittelt. Eine solche regulatorische Sequenz kann stromaufwärts des Transkriptionsinitiationspunkts eines Minimalpromotors, aber auch stromabwärts davon wie beispielsweise in einer transkribierten aber nicht translatierten Leader-Sequenz oder innerhalb eines Introns lokalisiert sein.

"Zur Verwendung als Haploideninduktor geeignet" meint, dass eine Pflanze in die Lage ist, befruchtete Samen, welche einen einfachen (haploiden) Chromosomensatz aufweisen, aus einer Kreuzung mit einer Pflanze derselben Gattung, vorzugsweise derselben Spezies, welche nicht die Eigenschaft eines Haploideninduktors aufweist, hervorzubringen. Die Verwendung als Haploideninduktor, angegeben als absolute Haploiden-Induktionsrate, meint, dass mindestens 0,1%, 0,2%, 0,3%, 0,4%, 0,5%, 0,6%, 0,7%, 0,8%, 0,9% oder 1%, bevorzugt mindestens 1,5%, 2%, 2,5%, 3%, 3,5%, 4%, 4,5% oder 5%, besonders bevorzugt mindestens 6%, 7%, 8%, 9%, 10%, 11%, 12%, 13%, 14% oder 15%, oder ganz besonders bevorzugt mindestens 20%, 25%, 30%, 35%, 40%, 45% oder 50% der befruchteten Samen einen haploiden Chromosomensatz aufweisen.

Ausgestaltungen und Ausführungsformen der vorliegenden Erfindung werden in exemplarischer Weise mit Bezug auf die angehängten Figuren und Sequenzen beschrieben:

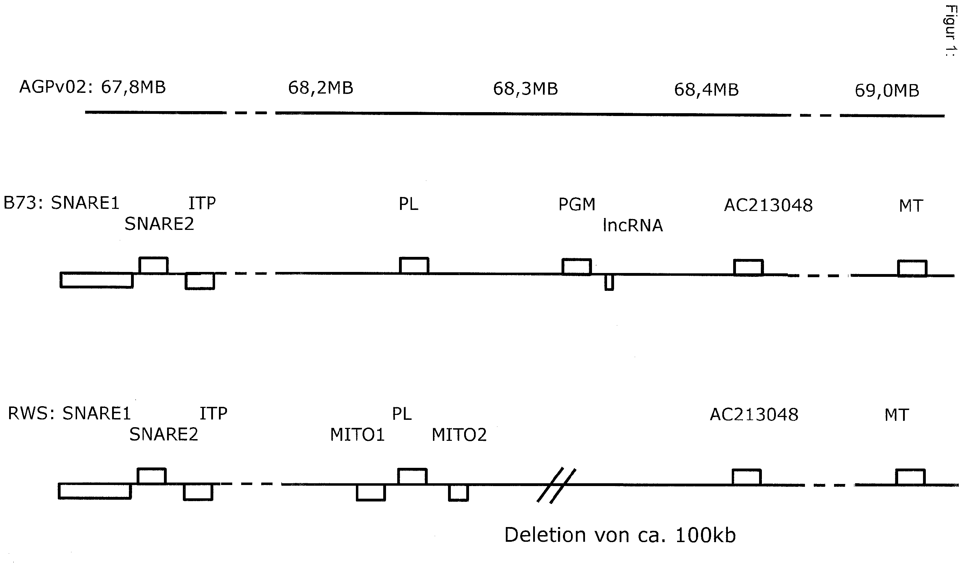

Figur 1: Genomische Anordnung der identifizierten Gene im Vergleich zu B73 (AGPv02): SNAREv 1 (GRMZM2G179789): erhöhte Expression in RWS-Pollen; SNAREv 2 (GRMZM2G412426): erhöhte Expression in RWS-Pollen; ITP (Inositol-1,4,5-Triphosphate-5-phosphatase) (GRMZM2G106834): reduzierte Expression in RWS Pollen; PL (Patatin-Phospholipase) (GRMZM2G471240): Polymorphismen in kodierender und regulierender Sequenz; PGM (Phosphoglyceratmutase) (GRMZM2G062320): Deletiert in RWS; IncRNA: Homolog zu PL: Deletiert in RWS; AC213048: Ankergen zum Vergleich der Sequenzen; MT (RNA-Methyltransferase) (GRMZM2G347808): Polymorphismen im regulatorischen Bereich. Die GRMZM-Namen beziehen sich auf die Annotation in AGPv02.

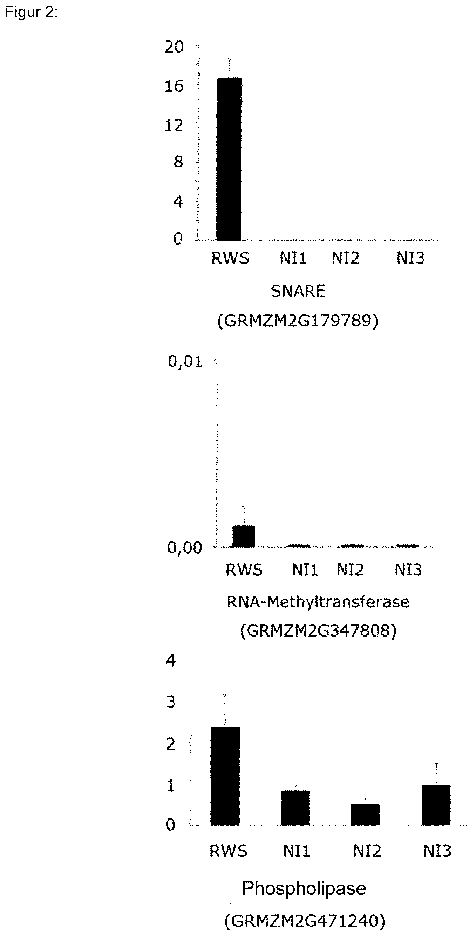

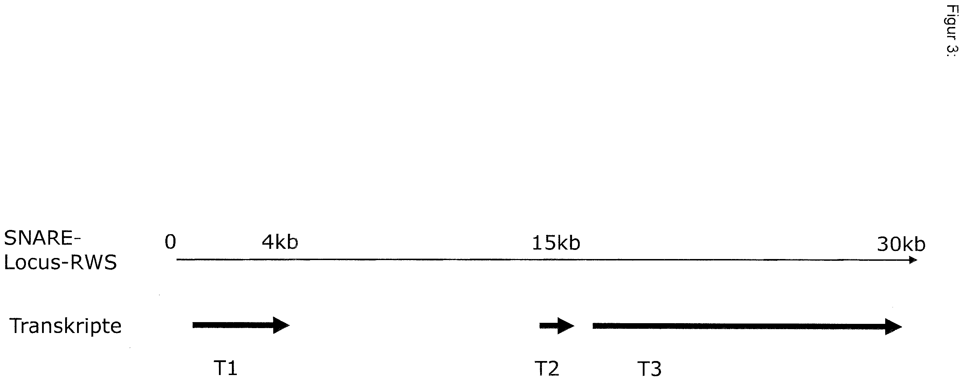

Figur 2: RT-PCR auf reifen Pollen in Induktor RWS und drei Nicht-Induktor-Kontrollen (NI1, NI2, NI3) über die Gene SNAREv 1, RNA-Methyltransferase und Patatin-Phospholipase.

QTL-Analyse und Identifikation von Kandidatengenen:
In dem Mais Haploideninduktor RWS, der auf den Induktor Stock6 (Literatur) zurückzuführen ist, wurde ein Haupt-QTL auf Chromosom 1 (bin 1.04) identifiziert und feinkartiert. Dieser QTL scheint einem QTL zu ähneln, der bereits im Stand der Technik beschrieben ist (Literatur). Ausgehend von diesen Arbeiten sollte der QTL in RWS verifiziert und molekular analysiert werden, um die zugrundeliegenden Gene zu identifizieren und funktional zu validieren. Eine QTL-Kartierungspopulation aus RWS x Kontrolle1 (maternaler Induktor x Nicht-Induktor) wurde auf Induktionsleistung getestet. Dabei konnte gezeigt werden, dass der bekannte QTL wahrscheinlich auch im Induktor RWS vorhanden ist. Weiterhin gelang es aber auch, eine starke Allelverschiebung zugunsten des Nicht-RWS (Kontrolle 1)-Allels aufzufinden.

Um den Locus molekular zu beschreiben, wurden verschiedene Sequenzieransätze auf DNA und auf RNA-Ebene gewählt. Aufgrund struktureller Unterschiede zwischen Induktoren und Referenzgenom B73 führen die klassischen referenzbasierten Sequenzieransätze nur in geringem Maße zum Erfolg. Ausgedehnte und aufwendige bioinformatorische Analysen ergaben, dass strukturelle Unterschiede dann über andere Technologien überprüft werden mußten.

Im Rahmen eines Sequence Capture-Ansatzes wurden ca. drei Megabasen um den identifizierten QTL in drei Stock6 abgeleiteten Induktoren inkl. RWS und fünf nicht-Induktor Kontrollensequenziert und auf Induktor-spezifische Polymorphismen wie Presence-Absence Variations, SNPs und InDels analysiert. Dabei wurden zunächst 16 Kandidatengene identifiziert, wovon durch Nachsequenzierung und Analyse von Expressionsdaten drei Gene bestätigt wurden: ein Gen, das für eine Antheren-spezifische Patatin-Phospholipase A2 kodiert, welche einen RWS-Induktor-spezifischer Haplotyp aufweist, ein Phosphoglycerat-Mutase-Gen, welches im Induktor RWS nicht vorhanden ist und ein RNA-Methyltransferase-Gen, welches eine Mutation in einer regulatorischen Sequenz aufweist.

Ferner wurden BAC-Banken für RWS, EMK (ein weiterer von Stock6 abgeleiteten Induktor) und Kontrolle 1 entwickelt und mit Sonden, verteilt über den identifizeirten QTL, gescreent. Für einen Ziel-Bereich von etwa 150 kb, welcher von Dong et al. 2013 im Induktor UH400 als möglicherweise Induktor-relevant erwähnt wurde, wurden BACs von RWS, Kontrolle 1 und EMK gefischt und sequenziert. Die BAC-Sequenzen wurden annotiert und mit umfangreichen Transkriptomdaten, welche für RWS, Kontrolle 1, EMK und B73 erstellt wurden, abgeglichen.

Im Ergebnis konnte die Deletion im Induktor hierbei bestätigt werden. Demnach fehlt den untersuchten maternalen Induktoren ein Bereich von ca. 100 kb zwischen 68,26 und 68,36 MB (AGP Version 2 der B73 Referenzsequenz) auf Chromosom 1. Weiterhin zeigen sich außerhalb des Ziel-Bereichs in den Induktoren eine Inversion in einer Gen-ähnlichen Region und ein großer repetitiver Sequenzabschnitt der mit dem Referenzgenom von B73 und mit Kontrolle1 nicht vergleichbar ist

Die bereits identifizierte Phospholipase ist trotz der Deletion in den Induktoren noch vorhanden, zeigt aber den bereits genannten stark von den Kontrollen abweichenden Haplotyp wie auch deutliche genetische Veränderungen im Promotorbereich. In Folge der Deletion ist in den Induktoren die bereits oben identifizierte Phosphoglycerat-Mutase nicht mehr vorhanden.

Weiterhin gelang es in der 100 kb-Deletion auch eine nicht kodierende RNA (IncRNA) zu identifizieren. Sie ist wie die Phospholipase Pollen-spezifisch exprimiert und zeigt außerdem eine Homologie von 82% zur identifizierten Phospholipase. Die Sequenz ist in sich komplementär, d.h., die IncRNA bildet eine Hairpin-Struktur. Die sehr hohe Expressionsrate, die signifikante Homologie zur Phospholipase und die niedrige SNP-Dichte, die durch Sangersequenzierung ermittelt wurde, weisen auf eine regulatorische Funktion dieser IncRNA für die Phospholipase hin. Theoretisch könnte von diesem Transkript auch eine 88 Aminosäuren lange, trunkierte Version des Phospholipase-Proteins translatiert werden.

Um zusätzlich zu den Polymorphismen auf DNA-Ebene auch Unterschiede im Expressionsniveau der identifizierten Gene aus der Region messen zu können, wurden RT-PCR- und RNASeq-Experimente durchgeführt. Neben RWP (einer Sublinie von RWS) als Induktor wurden drei genetisch sehr verschiedene Kontrolllinien eingesetzt. Von diesen Pflanzen wurde Pollen geerntet, Antheren ohne Pollen und Embryonen von 6-7 Tagen nach der Bestäubung von Selbstungen oder Kreuzungen. Hierbei zeigte die Phospholipase eine leicht erhöhte Expression im Pollen von RWP. Die Methyltransferase zeigt eine schwache Expression im Pollen von RWP und keine Expression im Pollen der Kontrollen. Die IncRNA ist pollenspezifisch exprimiert und fehlt, wie erwartet auch in RWP.

Zusätzlich wurde RNASeq auf Pollen des gleichen Materials angewendet, um vorstehende Ergebnisse weiter zu verfiizieren. Hierbei wurden unerwarteterweise, neue Kandidatengene außerhalb, aber nahe der oben genannten feinkartieren Region identifiziert, welche bisher wahrscheinlich aufgrund technischer Limitierung des SeqCapture Ansatzes nicht gefunden werden konnten. Etwa 400 kb stromaufwärts der identifizierten Phospholipase aus der feinkartieren Region liegt ein Genkomplex, welcher im Pollen von RWP deutlich unterschiedlich (mindestens Faktor 2) im Vergleich zu den Kontrollen exprimiert wird. Dieser Genkomplex enthält drei Gene: zwei als SNAREv-Gene annotierte Gene, welche zu einander eine hohe Homologie aufweisen und in RWP überexprimiert sind und ein Gen, das als Inositol-1,4,5-Trisphosphat-5-Phosphatase annotiert ist und dessen Expression in RWP reduziert ist. Klonierte Transkripte dieser Gene weichen zum Teil deutlich von der öffentlichen Annotation ob, so dass sie auch Proteine mit abweichenden Funktionen kodieren können, oder auch als IncRNAs funktionieren können. Bereits die SequenceCapture Daten dieser Region zeigen, dass es deutlich strukturelle Abweichungen zwischen Induktoren, Kontrollen und Referenzgenom gibt, sodass das Inositol-1,4,5-Trisphosphat-5-Phosphatase-Gen, wie es in B73 annotiert ist, in den Induktoren nicht vollständig amplifiziert werden konnte. Auch die Isolierung einer cDNA aus einem der SNARE-gene (GRMZM2G179789) weist auf komplexe strukturelle Änderungen in den Induktoren hin, da ein Teil der cDNA dem Plus-Strang und ein Teil dem Minus-Strang der Referenz entspricht.

Weiterhin findet sich in diesem Genkomplex auch ein bisher unbekanntes Gen, welches eine long non coding RNA kodiert. Dieses Gen teilt sich mit der identifizierten Inositol-1,4,5-Trisphosphat-5-Phosphatase einen gemeinsamen Transkriptionsstart, wird jedoch vom Gegenstrang angeschrieben.

### Gen-Funktionalitäten

Somit konnten insgesamt sechs Gene identifiziert werden, welche von Bedeutung für die *in vivo* Haploiden-Induktion oder die *in vivo* Haploiden-Induktionsleistung in Mais sein könnten.

Darunter vier Gene, welche für das Pollenschlauch-Wachtum von besonderer Bedeutung sind:
Die zwei SNAREv-Gene, kodieren für Proteine, welche bekanntermaßen am Vesikel-Transport beteiligt sind (Literatur). So konnten in der Modellpflanze *Arabidopsis thaliana* bereits SNAREv-Proteine an der Spitze des Pollenschlauches nachgewiesen werden, wo sie in den Transport von Phospholipiden und Pectinen involviert sind (Literatur). Die in den untersuchten Mais-Induktoren beobachtete Überexpression der SNAREv-Proteine würde zu erhöhtem Pollenschlauchwachstum führen.

Dass auch die Phospholipase A2 das Pollenschlauch-Wachtum deutlich beeinflusst, konnte in der Modellpflanze *Nicotiana tabacum* gezeigt werden. Die Inhibierung von Phospholipase A2 führt demnach zu einem Unterdrücken des Pollenschlauchwachstums (Kim et al., 2011). In den untersuchten Mais-Induktoren kann das Fehlen der identifizierten IncRNA mit signifikanter Homologie zur Phospholipase zu einer Reduzierung der Expressions- oder Translationsrate des Phopholipase-Gens führen, was die Wachstumsgeschwindigkeit des Pollenschlauchs beschleunigen würde.

In einer Knock-out Mutante der Inositol-Polyphosphate-5-Phosphatase in *Arabidopsis thaliana* zeigte sich, dass der Pollenschlauch ungehemmt wächst. In den untersuchten Mais-Induktoren kann das verminderte Expressionslevel der Inositol-1,4,5-Trisphosphat-5-Phosphatase somit ebenfalls zu einem beschleunigten Pollenschlauchwachstum führen. Die identifizierte, zur Inositol-1,4,5-Trisphosphat-5-Phosphatase zugehörige IncRNA könnte hierbei eine regulatorische Wirkung auf die Expressionrate haben.

Somit zeigen die untersuchten Mais-Induktoren eine geänderte Regulation/Expressionsrate der vier Gene im Vergleich zu Nicht-Induktoren. Diese Störung sollte zu einem deutlich schnelleren Pollenschlauchwachstum führen, was eine Entkoppluung des Transports der generativen Zellen im Pollenschlauch mit dessen Wachstum zu Folge hat. Als Folge davon kann es zu einer unvollständigen oder fehlerhaften Befruchtung mit anschließender Chromosomeneliminierung kommen.

Bekanntermaßen spielen aktive Centromere eine Schlüsselrolle bei der Chromsomenverteilung und werden über Chromatin-Modifikationen auf DNA- oder Histon-Ebene, außerdem durch Transkription, RNA-Wechselwirkungen und RNA Bindung, charakterisiert und modifiziert. Eine Änderung in der Regulation des Methyltransferase-Gens kann die Aktivität der Induktor-Centromere während der frühen Embryogenese beeinflussen, was letztendlich zur Eliminierung des Induktorgenoms im frühen Kornentwicklungsstadium führt.

In den untersuchten Induktoren konnte gezeigt werden, dass das Phophoglycerat-Mutase-Gen nicht mehr vorhanden ist. Das Fehlen des Gens kann den Energiestoffwechsel des Pollens beeinträchtigen und damit Auswirkungen auf die Befruchtung haben.

Zuständig für den Effekt der Haploiden-Induktion kann jedes Gen einzeln, oder jede Kombination von den Genen sein.

### Erstellung neuer in vivo Haploiden-Induktoren

Um einen neuen Induktor in anderen Kulturarten oder Mais-Nicht-Induktorgenotypen zu entwickeln oder die Induktionsleistung eines Induktorgenotyps zu erhöhen, ist folgendermaßen vorzugehen:
Identifikation der entsprechenden Gene in anderen Kulturarten oder Mais-Nicht-Induktorgenotypen: Die pollenspezifischen Patatin-Phospholipasen sind in einkeimblättrigen Pflanzen wie Mais, Reis, Weizen, Roggen oder Gerste stark konserviert und daher sind Homologe dazu leicht zu identifizieren. Regulatorische IncRNAs dagegen fehlen in den meisten einkeimblättrigen Pflanzen. Falls sie jedoch vorhanden sind, könnne sie ebenfalls anhand signifikanter Homologien, wie sie auch in den untersuchten Mais-Induktoren auftraten, aufgefunden werden. In zweikeimblättrigen Pflanzen übernehmen andere Phospholipase-Typen die entsprechenden Aufgaben im Pollenschlauchwachstum. Um diese zu identifizieren sind RNA-Banken von Pollen oder Pollenschläuchen zu erstellen und auf die spezifische Phospholipase der vorliegenden Erfindung zu screenen.

Die SNAREv-Gene und das Methyltransferase-Gen müssen nicht pollenspezifisch sein. So ist beispielsweise eines der identifizierten SNAREv-Gene (SNAREv 1) in Mais auch nicht pollenspezifisch exprimiert ist. Im Wildtyp-Pollen wird SNAREv 1 gar nicht exprimiert. In annotierten Genomen kann man über BLASTP und dem funktionalen Bereich eines SNAREv Proteins homologe Gene identifizieren. In nicht annotierten Genomen müsste man RNASeq Daten annotieren und nach SNARE-Genen selektieren.

Homologe Inositol-1,4,5-Trisphosphat-5-Phosphatasen und Phosphoglyceratmutasen müssen im Pollen exprimiert sein, um sie als Kandidatengene einsetzen zu können. Die identifikation kann wie oben erfolgen über BLASTP und anschließender RT-PCR in Pollen oder über Annotation von RNASeq Daten auf Pollen.

Manipulation der Kandidatengene: Mögliche Induktoren oder eine gesteigerte Induktionsleistung können durch transgene Expression der oben identifizierten Phospholipasen und/oder SNAREs und/oder Methyltransferase und/oder Phosphoglycerat-Mutasen und/oder IncRNAs erziet werden. Hierfür würde man die entsprechen Gene inklusive ihrer Promotoren aus der Induktorlinie RWP klonieren. Diese Gene würden in einen geeigneten Transformationsvektor kloniert und in die gewünschte Pflanze transformiert werden.

Die Pollen exprimierte Inositol-1,4,5-Trisphosphat-5-Phosphatase kann zusätzlich oder ausschließlich z.B. über RNAi in ihrer Aktivität reduziert werden. Hierfür sind beispielsweise Hairpin-Konstrukte herzustellen, welche dann inklusive eines geeigneten Promotors und Terminators, welche eine Transkription des Hairpin-Konstruktes vor oder zum Zeitpunkt der Pollenausbildung erlauben. Diese Hairpin-Konstrukte würden in einen geeigneten Transformationsvektor kloniert und in die gewünschte Pflanze transformiert werden.

Alternativ oder zusätzlich kann man über TILLING, Transposon-Mutagenese oder anderer Mutagenese-Verfahren oder ,Genome Editing' Pflanzen mit Mutationen z.B. in den identifizierten Genen erzeugen, die die Phospholipase und/oder SNAREs und/oder Methyltransferase stabilisieren, die Expression verstärken oder die Aktivität erhöhen. Hilfreich dazu können Strukturanalysen über Sekundär- und Tertiärstruktur der mutierten Proteine sein, die z.B auf dichtere Strukturen und damit weniger Angriffspunkte für Proteasen hinweisen. Außerdem können die Bereiche der Proteine betrachtet werden, die für Ubiquitinwechselwirkungen eine Rolle spielen. Mutanten im aktiven Zentrum des Gens können direkt auf ihre Aktivität getestet werden. Um die Aktivität der Inositol-1,4,5-Triphosphat-5-Phosphatase oder der Phosphoglycerat-Mutase zu manipulieren, muss man nach Knockout-Mutanten oder nach Mutanten suchen, die die Aktivität des Gens verringern.

Ferner kann man einen Stock6 abgeleiteten Induktor verbessern. Dies ist über den oben beschriebenen transgenen Ansatz wie auch über das Einbringen von Mutationen in den identifizierten Kandidatengenen möglich. Zusätzlich wäre es möglich, weitere Kopien der Gene im Genom, soweit sie im Pollen exprimiert sind, über transgene oder nicht-transgene Ansätze zu manipulieren.

Test der Induktionsleistung: Um die Induktionsleistung eines potentiellen Induktors zu testen, gibt es zum Beispiel die folgenden Möglichkeiten:
1. Bestäubung einer Linie mit einem visuellen rezessiven Marker (für Mais z.B. glossy (Bordes et al., 1997) oder liguless (Sylvester et al., 1990). Nachkommen, die dieses Merkmal ausprägen, werden über Flowzytometrie auf Haploidie geprüft.
2. Bestäubung einer Linie, die sich genetisch vom Induktor möglichst über mehrere Marker unterscheidet. Einsatz dieser Marker, um homozygote Pflanzen zu identifizieren. Diese Pflanzen werden über Flowzytometrie auf Haploidie geprüft.

### Referenzen

Barret, P., Brinkmann, M., & Beckert, M. (2008). A major locus expressed in the male gametophyte with incomplete penetrance is responsible for in situ gynogenesis in maize. Theoretical and Applied Genetics, 117(4), 581-594.
Bordes, J., de Vaulx, R. D., Lapierre, A., & Pollacsek, M. (1997). Haplodiploidization of maize (Zea mays L) through induced gynogenesis assisted by glossy markers and its use in breeding. Agronomie, 17(5), 291-297.
Chen, L., Tu, Z., Hussain, J., Cong, L., Yan, Y., Jin, L., ... & He, G. (2010). Isolation and heterologous transformation analysis of a pollen-specific promoter from wheat (Triticum aestivum L.). Molecular biology reports, 37(2), 737-744.
Chevalier, B. S., Kortemme, T., Chadsey, M. S., Baker, D., Monnat Jr, R. J., & Stoddard, B. L. (2002). Design, activity, and structure of a highly specific artificial endonuclease. Molecular cell, 10(4), 895-905.
Coe, E. H. (1959). A line of maize with high haploid frequency. American Naturalist, 381-382.
Das, L., & Martienssen, R. (1995). Site-selected transposon mutagenesis at the hcf106 locus in maize. The Plant Cell Online, 7(3), 287-294.
Deimling, S., Röber, F. K., Geiger, H. H. (1997). Methodik und Genetik der in-vivo-Haploideninduktion bei Mais. Vortr. Pflanzenzüchtung 38: 203-224.
Depicker, A., Stachel, S., Dhaese, P., Zambryski, P., & Goodman, H. M. (1981). Nopaline synthase: transcript mapping and DNA sequence. Journal of molecular and applied genetics, 1(6), 561-573.
Dong, X., Xu, X., Li, L., Liu, C., Tian, X., Li, W., & Chen, S. (2014). Marker-assisted selection and evaluation of high oil in vivo haploid inducers in maize. Molecular Breeding, 1-12.
Dong, X., Xu, X., Miao, J., Li, L., Zhang, D., Mi, X., ... & Chen, S. (2013). Fine mapping of qhir1 influencing in vivo haploid induction in maize. Theoretical and Applied Genetics, 126(7), 1713-1720.
Fire, A., Xu, S., Montgomery, M. K., Kostas, S. A., Driver, S. E., & Mello, C. C. (1998). Potent and specific genetic interference by double-stranded RNA in Caenorhabditis elegans. nature, 391(6669), 806-811.
Gaj, T., Gersbach, C. A., & Barbas III, C. F. (2013). ZFN, TALEN, and CRISPR/Cas-based methods for genome engineering. Trends in biotechnology, 31(7), 397-405.
Gurr, S. J., & Rushton, P. J. (2005). Engineering plants with increased disease resistance: what are we going to express?. TRENDS in Biotechnology, 23(6), 275-282.
Kato, N., He, H., & Steger, A. P. (2010). A systems model of vesicle trafficking in Arabidopsis pollen tubes. Plant physiology, 152(2), 590-601.
Kim, H. J., Ok, S. H., Bahn, S. C., Jang, J., Oh, S. A., Park, S. K., ... & Shin, J. S. (2011). Endoplasmic reticulum-and golgi-localized phospholipase A2 plays critical roles in Arabidopsis pollen development and germination. The Plant Cell Online, 23(1), 94-110.
Lloyd, A., Plaisier, C. L., Carroll, D., & Drews, G. N. (2005). Targeted mutagenesis using zincfinger nucleases in Arabidopsis. Proceedings of the National Academy of Sciences of the United States of America, 102(6), 2232-2237.
McCarty, D. R., Mark Settles, A., Suzuki, M., Tan, B. C., Latshaw, S., Porch, T., ... & Curtis Hannah, L. (2005). Steady-state transposon mutagenesis in inbred maize. The Plant Journal, 44(1), 52-61.
Odell, J. T., Nagy, F., & Chua, N. H. (1985). Identification of DNA sequences required for activity of the cauliflower mosaic virus 35S promoter.
Prigge, V., Xu, X., Li, L., Babu, R., Chen, S., Atlin, G. N., & Melchinger, A. E. (2012). New insights into the genetics of in vivo induction of maternal haploids, the backbone of doubled haploid technology in maize. Genetics, 190(2), 781-793.
Ravi, M., & Chan, S. W. (2010). Haploid plants produced by centromere-mediated genome elimination. Nature, 464(7288), 615-618.
Röber, F. K., Gordillo, G. A., & Geiger, H. H. (2005). In vivo haploid induction in maizeperformance of new inducers and significance of doubled haploid lines in hybrid breeding. Maydica, 50(3/4), 275.
Sambrook, J., Russell, D. W., & Russell, D. W. (2001). Molecular cloning: a laboratory manual (3-volume set) (Vol. 999). Cold Spring Harbor, New York:: Cold spring harbor laboratory press.
Shibuya, K., Fukushima, S., & Takatsuji, H. (2009). RNA-directed DNA methylation induces transcriptional activation in plants. Proceedings of the National Academy of Sciences, 106(5), 1660-1665.
Silva, G., Poirot, L., Galetto, R., Smith, J., Montoya, G., & Duchateau, P. (2011). Meganucleases and other tools for targeted genome engineering: perspectives and challenges for gene therapy. Current gene therapy, 11(1), 11.
Sylvester, A. W., Cande, W. Z., & Freeling, M. (1990). Division and differentiation during normal and liguleless-1 maize leaf development. Development, 110(3), 985-1000.
Till, B. J., Reynolds, S. H., Weil, C., Springer, N., Burtner, C., Young, K., ... & Henikoff, S. (2004). Discovery of induced point mutations in maize genes by TILLING. BMC Plant Biology, 4(1), 12.
Twell, D., Yamaguchi, J., Wing, R. A., Ushiba, J., & McCormick, S. (1991). Promoter analysis of genes that are coordinately expressed during pollen development reveals pollen-specific enhancer sequences and shared regulatory elements. Genes & development, 5(3), 496-507.
Venter, M. (2007). Synthetic promoters: genetic control through< i> cis</i> engineering. Trends in plant science, 12(3), 118-124.
Wang, Y., Chu, Y. J., & Xue, H. W. (2012). Inositol polyphosphate 5-phosphatase-controlled Ins (1, 4, 5) P3/Ca2+ is crucial for maintaining pollen dormancy and regulating early germination of pollen. Development, 139(12), 2221-2233.
Zhao, Y., Zhao, Q., Ao, G., & Yu, J. (2006). Characterization and functional analysis of a pollen-specific gene st901 in Solanum tuberosum. Planta, 224(2), 405-412.
WO/2010/079430 (Bonas et al.) Modular DNA-binding domains and methods of use WO/2011/072246 (Regents of the University of Minnesota) TAL effector-mediated DNA modification.
WO 2012/030893 (Monsanto Technology LLC) Molecular markers associated with haploid induction in *Zea mays.*

## Patentansprüche

1. Nukleinsäure, welche nach Transkription oder nach Expression in einer Pflanze geeignet ist, die Eigenschaft eines Haploideninduktors zu vermitteln oder die Induktionsleistung eines Haploideninduktors zu erhöhen, **dadurch gekennzeichnet, dass** die Nukleinsäure eine Nukleotidsequenz umfasst, die
(i) die Sequenz gemäß einer der SEQ ID NO: 9, 49 oder 52 aufweist, oder
(ii) die kodierende Sequenz gemäß einer der SEQ ID NO: 11, 50 oder 53 oder ein funktionelles Fragment der SEQ ID NO: 11, welches ein Abschnitt der SEQ ID NO: 11 ist und mindestens 350 aufeinanderfolgende Nukleotide der SEQ ID NO: 11 umfasst, aufweist, oder
(iii) zu einer Sequenz aus SEQ ID NO: 9 komplementär ist, oder
(iv) mit einer Sequenz aus SEQ ID NO: 9 zu mindestens 90% identisch ist, oder
(v) für ein Protein mit der Aminosäuresequenz ausgewählt aus SEQ ID NO: 23, 51 oder 54 kodiert.

2. Vektor, welcher die Nukleinsäure gemäß Anspruch 1 umfasst.

3. Wirtszelle, welche die Nukleinsäure gemäß Anspruch 1 oder den Vektor gemäß Anspruch 2 umfasst.

4. Transgene Pflanzenzelle, welche die Nukleinsäure gemäß Anspruch 1 als Transgen oder den Vektor gemäß Anspruch 2 umfasst.

5. Transgene Pflanze oder ein Samen davon, welche(r) eine Pflanzenzelle gemäß Anspruch 4 umfassen.

6. Verfahren zur Herstellung einer Pflanze, welche zur Verwendung als Haploideninduktor geeignet ist, umfassend die folgenden Schritte:
A) Mutagenisieren von Pflanzenzellen und anschließendem Regenerieren von Pflanzen aus den mutagenisierten Pflanzenzellen oder Mutagenisieren von Pflanzen, und
B) Identifizieren einer Pflanze aus A), welche in einer endogenen DNA-Sequenz, welche mit einer Nukleinsäure, umfassend eine Nukleotidsequenz, die
(i) die Sequenz gemäß SEQ ID NO: 8 aufweist, oder
(ii) die kodierende Sequenz gemäß der SEQ ID NO: 10 aufweist, oder
(iii) zu einer Sequenz aus (i) oder (ii) komplementär ist, oder
(iv) mit einer Sequenz aus (i) oder (ii) zu mindestens 80% identisch ist, oder
(v) für ein Protein mit der Aminosäuresequenz ausgewählt aus SEQ ID NO: 21 oder 22, einen funktionellen Teil der SEQ ID NO: 21 oder 22, welcher über eine Länge von mindestens 60% eine identische Aminosäuresequenz aufweist, kodiert,
oder mit der Nukleinsäure gemäß Anspruch 1 identisch ist, oder in einer regulatorischen Sequenz der endogenen DNA-Sequenz mindestens eine Mutation aufweist, die eine Änderung der Transkriptions- oder Expressionsrate der endogenen DNA-Sequenz in der identifizierten Pflanze im Vergleich mit einer nicht-mutagenisierten Wildtyp-Pflanze oder eine Änderung der Aktivität oder Stabilität eines durch die endogene DNA-Sequenz kodierten Proteins oder Polypeptids in der identifizierten Pflanze im Vergleich mit einer nicht-mutagenisierten Wildtyp-Pflanze bewirkt,
wobei die mindestens eine Mutation verursacht, dass in der identifizierten Pflanze die Eigenschaft eines Haploideninduktors vermittelt wird oder die Induktionsleistung eines Haploideninduktors erhöht wird.

7. Pflanze oder Samen der Pflanze, hergestellt durch das Verfahren gemäß Anspruch 6, wobei die mindestens eine Mutation eine Veränderung in der kodierenden Sequenz der SEQ ID NO: 8 oder 9, welche einen Aminosäureaustausch zwischen den Aminosäurepositionen 74 und 78 der SEQ ID NO: 21, 22 oder 23 verursacht, ist.

8. Pflanze oder Samen der Pflanze gemäß Anspruch 7 oder hergestellt durch das Verfahren gemäß Anspruch 6, welche die Sequenz gemäß einer der SEQ ID NO: 49 oder 52, oder die kodierende Sequenz gemäß einer der SEQ ID NO: 50 oder 53 umfasst.

9. Verfahren zur Isolation einer Nukleinsäure, die in einer Pflanze die Eigenschaft eines Haploideninduktors vermittelt oder die Induktionsleistung eines Haploideninduktors erhöht, umfassend die folgenden Schritte:
A) Herstellung einer Pflanze nach dem Verfahren gemäß Anspruch 6 oder Bereitstellung einer Pflanze gemäß Anspruch 7 oder 8,
B) Isolieren einer Nukleinsäure, welche die endogene DNA-Sequenz mit der mindestens einen Mutation umfasst, aus dem Genom der Pflanze aus A).

10. Verfahren zur Herstellung einer transgenen Pflanze, welche zur Verwendung als Haploideninduktor geeignet ist, umfassend die folgenden Schritte:
A) Bereitstellen der Nukleinsäure gemäß Anspruch 1 oder des Vektors gemäß Anspruch 2,
B) Transformieren von Pflanzenzellen durch Einbringen der Nukleinsäure oder des Vektors aus A),
C) Regenerieren transgener Pflanzen aus den transformierten Pflanzenzellen aus B), und
D) Identifizieren einer transgenen Pflanze aus C), in welcher durch ein geändertes Expressionsmuster die Eigenschaft eines Haploideninduktors vermittelt wird oder die Induktionsleistung eines Haploideninduktors erhöht wird.

11. Verwendung der Nukleinsäure gemäß Anspruch 1 oder des Vektors gemäß Anspruch 2 in einer Pflanze zur Vermittlung der Eigenschaft eines Haploideninduktors oder zur Erhöhung der Induktionsleistung eines Haploideninduktors oder zur Herstellung einer Pflanze oder einer transgenen Pflanze, welche zur Verwendung als Haploideninduktor geeignet ist.

12. Pflanze umfassend eine Nukleinsäure die
(i) die Sequenz gemäß einer der SEQ ID NO: 49 oder 52 aufweist, oder
(ii) die kodierende Sequenz gemäß einer der SEQ ID NO: 50 oder 53 aufweist, oder
(iii) für ein Protein mit der Aminosäuresequenz ausgewählt aus SEQ ID NO: 51 oder 54 kodiert.

13. Pflanze umfassend eine DNA die die Sequenz gemäß SEQ ID NO: 11 aufweist.

## Claims

1. A nucleic acid which, after transcription or after expression in a plant, is capable of mediating the property of a haploid inducer or of increasing the induction capacity of a haploid inducer, **characterized in that** the nucleic acid comprises a nucleotide sequence which
(i) has the sequence according to one of SEQ ID NO: 9, 49 or 52, or
(ii) has the coding sequence according to one of SEQ ID NO: 11, 50 or 53 or a functional fragment of SEQ ID NO: 11 which is a section of SEQ ID NO: 11 and comprises at least 350 consecutive nucleotides of SEQ ID NO: 11, or
(iii) is complementary to a sequence of SEQ ID NO: 9, or
(iv) is at least 90% identical to a sequence of SEQ ID NO: 9, or
(v) encodes a protein having the amino acid sequence selected from SEQ ID NO: 23, 51 or 54.

2. A vector comprising the nucleic acid according to claim 1.

3. A host cell comprising the nucleic acid according to claim 1 or the vector according to claim 2.

4. A transgenic plant cell comprising the nucleic acid according to claim 1 as transgene or the vector according to claim 2.

5. A transgenic plant or a seed thereof comprising a plant cell according to claim 4.

6. A method of producing a plant suitable for use as a haploid inducer comprising the following steps:
A) Mutagenizing plant cells and subsequently regenerating plants from the mutagenized plant cells or mutagenizing plants, and
B) Identifying a plant of A) which in an endogenous DNA sequence which is identical to a nucleic acid comprising a nucleotide sequence which
(i) has the sequence according to SEQ ID NO: 8, or
(ii) has the coding sequence according to SEQ ID NO: 10, or
(iii) is complementary to a sequence of (i) or (ii), or
(iv) is at least 80% identical to a sequence of (i) or (ii), or
(v) encodes for a protein having the amino acid sequence selected from SEQ ID NO: 21 or 22, a functional part of SEQ ID NO: 21 or 22 which has an identical amino acid sequence over a length of at least 60%,
or to the nucleic acid according to claim 1, or in a regulatory sequence of the endogenous DNA sequence has at least one mutation which causes a change in the transcription or expression rate of the endogenous DNA sequence in the identified plant in comparison to a non-mutagenized wild type plant or a change in the activity or stability of a protein or polypeptide, encoded by the endogenous DNA sequence, in the identified plant in comparison to a non-mutagenized wild type plant,
wherein the at least one mutation causes the property of a haploid inducer to be imparted in the identified plant or the induction capacity of a haploid inducer to be increased.

7. A plant or a seed of the plant produced by the method according to claim 6, wherein the at least one mutation is a change in the coding sequence of SEQ ID NO: 8 or 9 which causes an amino acid substitution between amino acid positions 74 and 78 of SEQ ID NO: 21, 22 or 23.

8. The plant or the seed of the plant according to claim 7 or produced by the method according to claim 6, comprising the sequence according to one of SEQ ID NO: 49 or 52, or the coding sequence according to one of SEQ ID NO: 50 or 53.

9. A method for isolating a nucleic acid which imparts the property of a haploid inducer or increases the inducing capacity of a haploid inducer in a plant, comprising the following steps:
A) Preparing a plant according to the method of claim 6 or providing a plant according to claim 7 or 8,
B) Isolating a nucleic acid comprising the endogenous DNA sequence with the at least one mutation from the genome of the plant of A).

10. A method of producing a transgenic plant suitable for use as a haploid inducer comprising the following steps:
A) Providing the nucleic acid according to claim 1 or the vector according to claim 2,
B) Transforming plant cells by inserting the nucleic acid or the vector of A),
C) Regenerating transgenic plants from the transformed plant cells of B), and
D) Identifying a transgenic plant from C) in which the property of a haploid inducer is imparted by an altered expression pattern or the induction capacity of a haploid inducer is increased.

11. A use of the nucleic acid according to claim 1 or the vector according to claim 2 in a plant for imparting the property of a haploid inducer or for increasing the induction capacity of a haploid inducer or for producing a plant or a transgenic plant which is suitable for use as a haploid inducer.

12. A plant comprising a nucleic acid which
(i) has the sequence according to one of SEQ ID NO: 49 or 52, or
(ii) has the coding sequence according to one of SEQ ID NO: 50 or 53, or
(iii) encodes a protein having the amino acid sequence selected from SEQ ID NO: 51 or 54.

13. Plant comprising a DNA having the sequence according to SEQ ID NO: 11.

## Revendications

1. Acide nucléique qui convient, après transcription ou après expression dans une plante, pour procurer la propriété d'un inducteur d'haploïdes ou pour augmenter la puissance d'induction d'un inducteur d'haploïdes, **caractérisé en ce que** l'acide nucléique comprend une séquence nucléotidique qui
(i) présente la séquence selon l'une des SEQ ID NO : 9, 49 ou 52 ou
(ii) présente la séquence codante selon l'une des SEQ ID NO :11, 50 ou 53 ou un fragment fonctionnel de la SEQ ID NO : 11, qui est une section de la SEQ ID NO: 11 et qui comprend au moins 350 nucléotides successifs de la SEQ ID NO : 11 ou
(iii) est complémentaire à une séquence de SEQ ID NO : 9 ou
(iv) est identique à raison d'au moins 90% à une séquence de SEQ ID NO : 9 ou
(v) code pour une protéine présentant la séquence d'acides aminés choisie parmi les SEQ ID NO : 23, 51 ou 54.

2. Vecteur qui comprend l'acide nucléique selon la revendication 1.

3. Cellule hôte qui comprend l'acide nucléique selon la revendication 1 ou le vecteur selon la revendication 2.

4. Cellule végétale transgénique qui comprend l'acide nucléique selon la revendication 1 en tant que transgène ou le vecteur selon la revendication 2.

5. Plante transgénique ou graine de celle-ci, qui comprend une cellule végétale selon la revendication 4.

6. Procédé de production d'une plante qui convient pour une utilisation en tant qu'inducteur d'haploïdes, comprenant les étapes suivantes :
A) mutagénisation de cellules végétales et régénération consécutive de plantes à partir des cellules végétales mutagénisées ou mutagénisation de plantes et
B) identification d'une plante de A), qui, dans une séquence d'ADN endogène, qui est identique à un acide nucléique comprenant une séquence nucléotidique qui
(i) présente la séquence selon la SEQ ID NO : 8 ou
(ii) présente la séquence codante selon la SEQ ID NO : 10 ou
(iii) est complémentaire à une séquence de (i) ou (ii) ou
(iv) est identique à raison d'au moins 80% à une séquence de (i) ou (ii) ou
(v) code pour une protéine présentant la séquence d'acides aminés choisie parmi les SEQ ID NO : 21 ou 22, une partie fonctionnelle des SEQ ID NO : 21 ou 22, qui présente une séquence d'acides aminés identique sur une longueur d'au moins 60%,
ou qui est identique à l'acide nucléique selon la revendication 1 ou présente, dans une séquence de régulation de la séquence d'ADN endogène, au moins une mutation qui provoque une modification du taux de transcription ou d'expression de la séquence d'ADN endogène dans la plage identifiée par rapport à une plante de type sauvage, non mutagénisée ou une modification de l'activité ou de la stabilité d'une protéine ou d'un polypeptide codée par la séquence d'ADN endogène dans la plante identifiée par rapport à une plante de type sauvage non mutagénisée,
ladite au moins une mutation entraînant que, dans la plante identifiée, la propriété d'un inducteur d'haploïdes est procurée ou la puissance d'induction d'un inducteur d'haploïdes est augmentée.

7. Plante ou graine de la plante, produite par le procédé selon la revendication 6,
ladite au moins une mutation étant une modification dans la séquence codante des SEQ ID NO : 8 ou 9, qui entraîne un échange d'acides aminés entre les positions d'acide aminé 74 et 78 des SEQ ID NO: 21, 22 ou 23.

8. Plante ou graine de la plante selon la revendication 7 ou produite par le procédé selon la revendication 6 qui comprend la séquence selon l'une des SEQ ID NO: 49 ou 52 ou la séquence codante selon l'une des SEQ ID NO : 50 ou 53.

9. Procédé d'isolement d'un acide nucléique qui procure, dans une plante, la propriété d'un inducteur d'haploïdes ou qui augmente la puissance d'induction d'un inducteur d'haploïdes, comprenant les étapes suivantes :
A) production d'une plante selon le procédé selon la revendication 6 ou mise à disposition d'une plante selon la revendication 7 ou 8,
B) isolement d'un acide nucléique qui comprend la séquence d'ADN endogène présentant ladite au moins une mutation, à partir du génome de la plante de A).

10. Procédé pour la production d'une plante transgénique qui convient pour une utilisation en tant qu'inducteur d'haploïdes, comprenant les étapes suivantes :
A) mise à disposition de l'acide nucléique selon la revendication 1 ou du vecteur selon la revendication 2,
B) transformation de cellules végétales par introduction de l'acide nucléique ou du vecteur de A),
C) régénération de plantes transgéniques à partir des cellules végétales transformées de B) et
D) identification d'une plante transgénique de C) dans laquelle la propriété d'un inducteur d'haploïdes est procurée ou la puissance d'induction d'un inducteur d'haploïdes est augmentée par un motif d'expression modifié.

11. Utilisation de l'acide nucléique selon la revendication 1 ou du vecteur selon la revendication 2 dans une plante afin de procurer la propriété d'un inducteur d'haploïdes ou d'augmenter la puissance d'induction d'un inducteur d'haploïdes ou de produire une plante ou une plante transgénique qui convient pour une utilisation comme inducteur d'haploïdes.

12. Plante comprenant un acide nucléique qui
(i) présente la séquence selon l'une des SEQ ID NO : 49 ou 52 ou
(ii) présente la séquence codante selon l'une des SEQ ID NO : 50 ou 53 ou
(iii) code pour une protéine présentant la séquence d'acides aminés choisie parmi les SEQ ID NO: 51 ou 54.

13. Plante comprenant un ADN qui présente la séquence selon la SEQ ID NO : 11.
